# EUROPEAN PATENT APPLICATION

(11) **EP 0 965 639 A1**
(43) Date of publication of application: **22.12.1999**
(21) Application number: 98110356.7
(22) Date of filing: 05.06.1998
(51) Int. Cl.: C12N 15/40, A61K 35/76, C12N 7/04, A61K 48/00, C12Q 1/70

(54) **Attenuated pestiviruses**

(71) Applicant: BOEHRINGER INGELHEIM VETMEDICA GMBH, 55216 Ingelheim (DE)
(72) Inventor: Meyers, Gregor, Dr., 72141 Walddorf-Häslach (DE)
(74) Representative: Laudien, Dieter, Dr.

(57) **Abstract**

This invention relates to attenuated pestiviruses characterised in that their enzymatic activity residing in glycoprotein E^{RNS} is inactivated, methods of preparing, using and detecting these.

## Description

### Field of the invention

The present invention relates to a method for attenuating pestiviruses by inactivating the ribonuclease activity (RNase activity) residing in glycoprotein E^{RNS}. The invention also relates to pestiviruses attenuated according to the invention, nucleic acids for preparing such pestiviruses, vaccines and pharmaceutical compositions comprising the attenuated pestiviruses of the invention. The invention further relates to methods for distinguishing between the attenuated viruses of the invention and pathogenic viruses.

### Background of the invention

Pestiviruses are causative agents of economically important diseases of animals in many countries worldwide. Presently known virus isolates have been grouped into three different species which together form one genus within the family Flaviviridae.
I Bovine viral diarrhea virus (BVDV) causes bovine viral diarrhea (BVD) and mucosal disease (MD) in cattle (Baker, 1987; Moennig and Plagemann, 1992; Thiel et al., 1996).
II Classical swine fever virus (CSFV), formerly named hog cholera virus, is responsible for classical swine fever (CSF) or hog cholera (HC) (Moennig and Plagemann, 1992; Thiel et al., 1996).
III Border disease virus (BDV) is typically found in sheep and causes border disease (BD). Symptoms similar to MD in cattle have also been described to occur after intrauterine infection of lambs with BDV (Moennig and Plagemann, 1992; Thiel et al., 1996).
An alternative classification of pestiviruses is provided by Becher et al. (1995) or others.
Pestiviruses are small enveloped viruses with a single stranded RNA genome of positive polarity lacking both 5' cap and 3' poly(A) sequences. The viral genome codes for a polyprotein of about 4000 amino acids giving rise to final cleavage products by co- and posttranslational processing involving cellular and viral proteases. The viral proteins are arranged in the polyprotein in the order NH₂-N^{pro}-C-E^{RNS}-E1-E2-p7-NS2-NS3-NS4A-NS4B-NS5A-NS5B-COOH (Rice, 1996). Protein C and the glycoproteins E^{RNS}, E1 and E2 represent structural components of the pestivirus virion (Thiel et al., 1991). E2 and to a lesser extent E^{RNS} were found to be targets for antibody neutralization (Donis et al., 1988; Paton et al., 1992; van Rijn et al., 1993; Weiland et al., 1990, 1992). E^{RNS} lacks a membrane anchor and is secreted in considerable amounts from the infected cells; this protein has been reported to exhibit RNase activity (Hulst et al., 1994; Schneider et al., 1993; Windisch et al., 1996). The function of this enzymatic activity for the viral life cycle is presently unknown. In the case of a CSFV vaccine strain experimental destruction of the RNase by site directed mutagenesis has been reported to result in a cytopathogenic virus that has growth characteristics in cell culture equivalent to wild type virus (Hulst et at., 1998). The enzymatic activity depends on the presence of two stretches of amino acids conserved between the pestivirus E^{RNS} and different known RNases of plant and fungal origin. Both of these conserved sequences contain a histidine residue (Schneider et al., 1993). Exchange of each of these residues against lysine in the E^{RNS} protein of a CSFV vaccine strain resulted in the destruction of RNase activity (Hulst et al., 1998). Introduction of these mutations into the genome of the CSFV vaccine strain did not influence viral viability or growth properties but led to a virus exhibiting a slightly cytopathogenic phenotype (Hulst et al., 1998).

Vaccines comprising attenuated or killed viruses or viral proteins expressed in heterologous expression systems have been generated for CSFV and BVDV and are presently used. The structural basis of the attenuation of these viruses used as life vaccines is not known. This leads to the risk of unpredictable revertants by backmutation or recombination subsequent to vaccination. On the other hand, the efficacy of inactivated vaccines or heterologously expressed viral proteins (subunit vaccines) in the induction of immunity is rather low.
In general, live vaccines with defined mutations as a basis for attenuation would allow to avoid the disadvantages of the present generation of vaccines. Potential targets for attenuating mutations in pestiviruses are not available at present.

A further advantage of said attenuating mutations lies in their molecular uniqueness which allows to use them as distinctive labels for an attenuated pestiviruses and to distinguish them from pestiviruses from the field.

Because of the importance of an effective and safe as well as detectable prophylaxis and treatment of pestiviral infections, there is a strong need for live and specifically attenuated vaccines with a high potential for induction of immunity as well as a defined basis of attenuation which can also be distinguished from pathogenic pestiviruses.

Therefore, the technical problem underlying the present invention is to provide specifically attenuated and detectably labeled pestiviruses for use as live attenuated vaccines with a high efficiency for the induction of immunity which, as a result of this method, can also be distinguished from pathogenic pestiviruses from the field.

### Disclosure of the invention

The solution to the above technical problem is achieved by the description and the embodiments characterized in the claims.
It has surprisingly been found that pestiviruses can be specifically attenuated by the inactivation of the RNase activity residing in glycoprotein E^{RNS}.
The attenuated pestiviruses now provide live vaccines of high immunogenicity. Therefore, in one aspect the present invention provides a live vaccine comprising a pestivirus, wherein the RNase activity residing in glycoprotein E^{RNS} is inactivated. The term "vaccine" as used herein refers to a pharmaceutical composition comprising at least one immunologically active component that induces an immunological response in an animal and possibly but not necessarily one or more additional components that enhance the immunological activity of said active component. A vaccine may additionally comprise further components typical to pharmaceutical compostions. The immunologically active component of a vaccine may comprise complete live organisms in either its original form or as attenuated organisms in a so called modified live vaccine (MLV) or organisms inactivated by appropriate methods in a so called killed vaccine (KV). In another form the immunologically active component of a vaccine may comprise appropriate elements of said organisms (subunit vaccines) whereby these elements are generated either by destroying the whole organism or the growth cultures of such organisms and subsequent purification steps yielding in the desired structure(s), or by synthetic processes induced by an appropriate manipulation of a suitable system like, but not restricted to bacteria, insects, mammalian or other species plus subsequent isolation and purification procedures or by induction of said synthetic processes in the animal needing a vaccine by direct incorporation of genetic material using suitable pharmaceutical compositions (polynucleotide vaccination). A vaccine may comprise one or simultaneously more than one of the elements described above.
Additional components to enhance the immune response are constituents commonly referred to as adjuvants, like e.g. aluminiumhydroxide, mineral or other oils or ancillary molecules added to the vaccine or generated by the body after the respective induction by such additional components, like but not restricted to interferons, interleukins or growth factors.
A "pharmaceutical composition" essentially consists of one or more ingredients capable of modifying physiological e.g. immunological functions of the organism it is administered to, or of organisms living in or on its surface like but not restricted to antibiotics or antiparasitics, as well as other constituents added to it in order to achieve certain other objectives like, but not limited to, processing traits, sterility, stability, feasibility to administer the composition via enteral or parenteral routes such as oral, intranasal, intravenous, intramuscular, subcutaneous, intradermal or other suitable route, tolerance after administration, controlled release properties.
A vaccine of the invention refers to a vaccine as defined above, wherein one immunologically active component is a pestivirus or of pestiviral origin.
The term "live vaccine" refers to a vaccine comprising a living, in particular, a living viral active component.
The term "pestivirus" as used herein refers to all pestiviruses, characterized by belonging to the same genus as BVDV, CSFV and BDV within the family Flaviviridae and by their expression of glycoprotein E^{RNS}. Of course, said term also refers to all pestiviruses as characterized by Becher et al. (1995) or others that express glycoprotein E^{RNS}. "RNase activity" as used herein refers to the ability of the glycoprotein E^{RNS} to hydrolyze RNA.

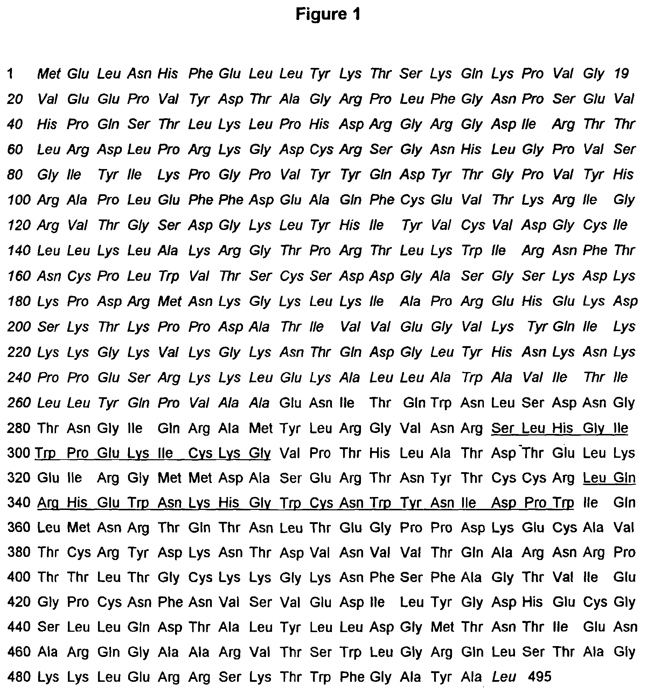

It should be noted that the term glycoprotein E0 is often used synonymously to glycoprotein E^{RNS} in publications.
The term "inactivation of the RNase activity residing in said glycoprotein" refers to the inability or reduced capability of a modified glycoprotein E^{RNS} to hydrolyze RNA as compared to the unmodified wild type of said glycoprotein E^{RNS}.
Inactivation of the RNase activity residing in glycoprotein E^{RNS} can be achieved by deletions and/or mutations of at least one amino acid of said glycoprotein as demonstrated herein and by Hulst et al. (1998). Therefore, in a preferred embodiment the present invention relates to live vaccines, wherein said RNase activity is inactivated by deletions and/or mutations of at least one amino acid of said glycoprotein.
It has been shown that the glycoprotein E^{RNS} forms a disulfide-bonded homodimer of about 97 kD, wherein each monomer consists of 227 amino acids corresponding to the amino acids 268 to 494 of the CSFV polyprotein as described by Rümenapf et al. (1993). The first 495 amino acids as expressed by the Alfort strain of CSFV are shown in figure 1 for reference purpose only. The genome sequence of the Alfort strain of CSFV is available in the GenBank/EMBL data library under accession number J04358; alternatively, the amino acid sequence for the BVDV strain CP7 can be accessed in the GenBank/EMBL data library (accession number U63479). Two regions of amino acids are highly conserved in glycoprotein E^{RNS} as well as in some plant and fungal RNase-active proteins (Schneider et al., 1993). These two regions are of particular importance to the RNase enzymatic activity. The first region consists of the region at the amino acids at position 295 to 307 and the second region consists of the amino acids at position 338 to 357 of said viral polyprotein as exemplified by figure 1 for the Alfort strain of CSFV (numbering according to the published deduced amino acid sequence of CSFV strain Alfort (Meyers et al., 1989). The amino acids of particular importance to the RNase activity as mentioned above are by no means limited to the exact position as defined for the Alfort strain of CSFV but are simply used in an exemplary manner to point out the preferred amino acids being at that position or corresponding to that position in other strains such as found in BVDV, BDV and pestiviruses in general since they are highly conserved. For pestiviruses other than the CSFV Alfort strain the numbering of the positions of the preferred amino acids is often different but an expert in the field of the molecular biology of pestiviruses will easily identify these preferred amino acids by their position relative to the highly conserved amino acids of said glycoprotein. As a consequence, the present invention relates in a more preferred embodiment to a vaccine of the invention, wherein said inactivating deletions and/or mutations are located at the amino acids at position 295 to 307 and/or position 338 to 357, as described in figure 1 for the CSFV Alfort strain in an exemplary manner or corresponding thereto in other strains, of said glycoprotein.
In a very preferred embodiment the present invention discloses that the inactivation of said RNase activity by deletion or mutation of the amino acid at position 346 of said glycoprotein leads to particularly useful live vaccines. Therefore, the present invention relates to vaccines according to the invention, wherein said Rnase activity is inactivated by deletion or mutation of the amino acid at position 346, as described in figure 1 for the CSFV Alfort strain in an exemplary manner or corresponding thereto in other strains, of said glycoprotein.
The present invention demonstrates that pestiviruses are viable and code for an E^{RNS} protein without RNase activity when the histidine residue at position 346 of the viral polyprotein (numbering according to the published sequence of CSFV Alfort/Tübingen (Meyers et al., 1989)), which represents one of the conserved putative active site residues of the E^{RNS} RNase, is deleted. It has also been demonstrated for this invention that the deletion of the respective histidine in the E^{RNS} of a BVD pestivirus (position 349, numbered according to the sequence of BVDV CP7 GenBank/EMBL data library (accession number U63479)) results in a viable virus in which the E^{RNS} glycoprotein has lost the RNase activity. In contrast to point mutations changing one amino acid into another, a deletion mutant is generally much more stable with respect to revertants. Infection of pigs with a mutant of the pathogenic CSFV Alfort/Tübingen expressing E^{RNS} with this deletion did not lead to fever or other typical clinical signs of CSFV infections whereas the infection with wild type virus resulted in fever, diarrhea, anorexia, apathy, and central nervous disorders. These pigs were killed in a moribund stage showing severe hemorrhages in the skin and internal organs 14 days post inoculation. The pigs infected with the mutant did not show viremia as tested on days 3, 5, 7, 10, 14 post infection while CSFV was easily isolated from blood samples derived from the pigs inoculated with wild type virus. The deletion mutant apparently replicated in the animals as indicated by the induction of neutralizing antibodies (see Example 4, Table 2). The immune response to the mutant virus was sufficient to permit to survive a lethal challenge with 2x10⁵ TCID₅₀ of the highly pathogenic infection with the CSFV strain Eystrup (König, 1994) which is heterologous to the Alfort strain. Moreover, the tested animals displayed no typical clinical signs for CSFV infection like fever, diarrhea, hemorrhages, anorexia after the challenge infection. This data demonstrates that infection of pigs with the deletion mutant induces an immune response sufficient for protection against a stringent challenge.
Therefore, in a most preferred embodiment the invention relates to vaccines according to the invention, wherein said RNase activity is inactivated by the deletion of the histidine residue at position 346, as described in figure 1 for the CSFV Alfort strain in an exemplary manner or corresponding thereto in other strains, of said glycoprotein.

In another aspect the present invention relates to attenuated pestiviruses, wherein the RNase activity residing in glycoprotein E^{RNS} is inactivated by deletions and/or mutations of at least one amino acid of said glycoprotein with the proviso that the amino acids at position 297 and/or 346 of said glycoprotein as described in figure 1 for CSFV are not lysine. A recombinant pestivirus, wherein amino acids at position 297 and/or 346 of said glycoprotein are lysine has been described by Hulst et al. in 1998. These particular pestiviruses demonstrated cytopathic effects in swine kidney cells. Up to now, there has been total unawareness of the surprising and innovative attenuating feature due to the inactivation of the E^{RNS} enzymatic activity.
In a preferred embodiment for the reasons stated above for vaccines the present invention also relates to pestiviruses according to the invention, wherein said RNase activity is inactivated by deletions and/or mutations located at the amino acids at position 295 to 307 and/or position 338 to 357, as described in figure 1 for the CSFV Alfort strain in an exemplary manner or corresponding thereto in other strains, of said glycoprotein.
In a more preferred embodiment for the reasons stated above for vaccines the present invention also relates to pestiviruses of the invention, wherein said RNase activity is inactivated by deletion or mutation of the amino acid at position 346, as described in figure 1 for the CSFV Alfort strain in an exemplary manner or corresponding thereto in other strains, of said glycoprotein.
In a most preferred embodiment for the reasons stated above for vaccines the present invention also relates to pestiviruses, wherein said RNase activity is inactivated by the deletion of the histidine residue at position 346, as described in figure 1 for the CSFV Alfort strain in an exemplary manner or corresponding thereto in other strains, of said glycoprotein.

The attenuated pestiviruses and active components of the vaccines of the present invention can easily be prepared by nucleic acid-modifying recombinant techniques resulting in the expression of a mutant amino acid sequence in glycoprotein E^{RNS}. Therefore, a further aspect of the present invention relates to nucleic acids coding for a glycoprotein E^{RNS}, wherein the RNase activity residing in said glycoprotein is inactivated by deletions and/or mutations of at least one amino acid of said glycoprotein with the proviso that the amino acids at position 297 and/or 346 of the glycoprotein as described in figure 1 for the CSFV Alfort strain are not lysine.
In a preferred embodiment the present invention relates, for reasons as mentioned above, to nucleic acids according to the invention, wherein said RNase activity is inactivated by deletions and/or mutations that are located at the amino acids at position 295 to 307 and/or position 338 to 357, as described in figure 1 for the CSFV Alfort strain in an exemplary manner or corresponding thereto in other strains, of said glycoprotein.
In a more preferred embodiment the present invention relates, for reasons as mentioned for vaccines, to nucleic acids according to the invention, wherein said RNase activity is inactivated by deletion or mutation of the amino acid at position 346, as described in figure 1 for the CSFV Alfort strain in an exemplary manner or corresponding thereto in other strains, of said glycoprotein.
In a most preferred embodiment the present invention relates to nucleic acids according to the invention, wherein said Rnase activity is inactivated by the deletion of the histidine residue at position 346, as described in figure 1 for the CSFV Alfort strain in an exemplary manner or corresponding thereto in other strains, of said glycoprotein.

The vaccines, attenuated pestiviruses, and/or nucleic acids according to the invention are particularly useful for the preparation of a pharmaceutical composition. In consequence, a further aspect of the present invention relates to pharmaceutical compositions comprising a vaccine according to the invention, and/or a pestivirus according to the invention, and/or a nucleotide sequence according to the invention.

An additional aspect of the present invention relates to a method of attenuation for pestiviruses. The invention provides a unique and unexpected method for attenuating pestiviruses characterized in that the RNase activity residing in glycoprotein E^{RNS} is inactivated.

The specifically attenuated pestiviruses are especially useful for the preparation of vaccines. Therefore, in a further additional aspect the present invention relates to methods for producing a specifically attenuated pestivirus vaccine characterized in that the Rnase activity residing in glycoprotein E^{RNS} is inactivated.

The inactivation of the RNase activity residing in glycoprotein E^{RNS} provides a surprising and new method for detectably labeling pestiviruses. In a further aspect the present invention provides a method for detectably labeling pestiviruses characterized in that the RNase activity residing in glycoprotein E^{RNS} is inactivated. The feature of absence of RNase activity residing in the glycoprotein E^{RNS} of pestiviruses of the invention now enables for detectably labeling these pestiviruses. Labeled and unlabeled pestiviruses or the E^{RNS} secreted from pestivirus infected cells in body fluids can clearly be distinguished by the absence or presence of RNase activity of the glycoproteins E^{RNS} upon isolation and assaying such enzymatic activity.
For pestiviruses inactivated in their RNase activity residing in glycoprotein E^{RNS} by deletion and/or mutation, a number of other techniques can be used. Such pestiviruses can easily be detected because of the structural consequences resulting from such deletions and/or mutations. For example, the sequence difference of the nuclic acid sequence of altered glycoprotein E^{RNS} is detectable by nucleic acid sequencing techniques; the altered protein sequence can be detected by specific monoclonal antibodies, that do not recognize unaltered proteins. Vice versa, it is also possible to detect the altered and thereby structurally labeled proteins by the absence of binding to specific monoclonal antibodies that recognize unaltered glycoproteins E^{RNS} under the proviso that the presence of pestiviruses can be established otherwise. And, of course, the deletions and/or mutations abrogating the RNase activity in the labeled viruses will result in different immune responses in animals when compared to the responses resulting from unlabeled pestivirus infections.
A preferred embodiment for all aspects referring to methods for attenuating pestiviruses, methods for producing a specifically attenuated pestivirus vaccine and methods for detectably labeling pestiviruses according to the invention are those methods relating to the inactivation of the glycoprotein E^{RNS}, wherein said RNase activity is inactivated by deletions and/or mutations of at least one amino acid of said glycoprotein.
A more preferred embodiment for all aspects referring to methods for attenuating pestiviruses, methods for producing a specifically attenuated pestivirus vaccine and methods for detectably labeling pestiviruses according to the invention are those methods relating to the inactivation of the glycoprotein E^{RNS}, wherein said deletions and/or mutations are located at the amino acids at position 295 to 307 and/or position 338 to 357, as described in figure 1 for the CSFV Alfort strain in an exemplary manner or corresponding thereto in other strains, of said glycoprotein.
A very preferred embodiment for all aspects referring to methods for attenuating pestiviruses, methods for producing a specifically attenuated pestivirus vaccine and methods for detectably labeling pestiviruses according to the invention are those methods relating to the inactivation of the glycoprotein E^{RNS}, wherein said RNase activity is inactivated by deletion or mutation of the amino acid at position 346, as described in figure 1 for the CSFV Alfort strain in an exemplary manner or corresponding thereto in other strains, of said glycoprotein.
A most preferred embodiment for all aspects referring to methods for attenuating pestiviruses, methods for producing a specifically attenuated pestivirus vaccine and methods for detectably labeling pestiviruses according to the invention are those methods relating to the inactivation of the glycoprotein E^{RNS}, wherein said RNase activity is inactivated by the deletion of the histidine residue at position 346, as described in figure 1 for the CSFV Alfort strain in an exemplary manner or corresponding thereto in other strains, of said glycoprotein.

The present invention provides vaccines and or other pharmaceutical compositions which are particularly useful for the prophylaxis and treatment of pestivirus infections in animals. Therefore, a further aspect of the present invention relates to methods for the prophylaxis and treatment of pestivirus infections in animals characterized in that a vaccine according to the invention or another pharmaceutical composition according to the invention is applied to an animal in need of such prophylaxis or treatment.

In a further aspect the present invention provides a process for the preparation of specifically attenuated pestiviruses characterized in that the RNase activity residing in glycoprotein E^{RNS} is inactivated.

In one aspect the present invention provides a process for the preparation of specifically labeled pestiviruses characterized in that the RNase activity residing in glycoprotein E^{RNS} is inactivated.

A preferred embodiment for all aspects referring to a process for the preparation of specifically attenuated pestiviruses, a process for the preparation of specifically labeled pestiviruses according to the invention are those processes relating to the inactivation of the glycoprotein E^{RNS}, wherein said RNase activity, is inactivated by deletions and/or mutations of at least one amino acid of said glycoprotein.
A more preferred embodiment for all aspects referring to a process for the preparation of specifically attenuated pestiviruses, a process for the preparation of specifically labeled pestiviruses according to the invention are those processes relating to the inactivation of the glycoprotein E^{RNS}, wherein said deletions and/or mutations are located at the amino acids at position 295 to 307 and/or position 338 to 357, as described in figure 1 for the CSFV Alfort strain in an exemplary manner or corresponding thereto in other strains, of said glycoprotein.
A very preferred embodiment for all aspects referring to a process for the preparation of specifically attenuated pestiviruses, a process for the preparation of specifically labeled pestiviruses according to the invention are those processes relating to the inactivation of the glycoprotein E^{RNS}, wherein said Rnase activity is inactivated by deletion or mutation of the amino acid at position 346, as described in figure 1 for the CSFV Alfort strain in an exemplary manner or corresponding thereto in other strains, of said glycoprotein.
A most preferred embodiment for all aspects referring to a process for the preparation of specifically attenuated pestiviruses, a process for the preparation of specifically labeled pestiviruses according to the invention are those processes relating to the inactivation of the glycoprotein E^{RNS}, wherein said RNase activity is inactivated by the deletion of the histidine residue at position 346, as described in figure 1 for the CSFV Alfort strain in an exemplary manner or corresponding thereto in other strains, of said glycoprotein.

The vaccines or other pharmaceutical compositions of the present invention are useful for the prophylaxis and treatment of pestivirus infections in animals.
Therefore, in one aspect the present invention relates to the use of a vaccine according to the invention for the prophylaxis and treatment of pestivirus infections in animals. In a further aspect the present invention relates to the use of a pharmaceutical composition according to the invention for the prophylaxis and treatment of pestivirus infections in animals.

Pestiviruses and/or nucleic acids according to the invention are useful active components of a pharmaceutical composition or a vaccine. Therefore, the present invention relates in a further aspect to the use of a pestivirus of the invention and/or a nucleic acid of the invention for the preparation of a vaccine or a pharmaceutical composition.

As mentioned above the inactivation of the RNase activity residing in glycoprotein E^{RNS} provides a surprising and new method for labeling pestiviruses.
As a consequence one aspect of the present invention relates to methods for distinguishing the detectably labeled pestiviruses according to the invention from unlabeled and possibly pathogenic pestiviruses. Such methods are especially useful for tracing the efficacy of labeled pestiviruses in animals. A vaccine treated animal will prove label-positive after obtaining a sample of such animal and assaying for said label. Unlabeled animals and especially unlabeled animals that prove pestivirus positive can be immediately separated, isolated or slaughtered to remove the imminent danger of spreading the pathogenic infection to other animals.
The present invention provides a method for detectably labeling pestiviruses characterized in that the RNase activity residing in glycoprotein E^{RNS} is inactivated. This feature of absence of RNase activity residing in the glycoprotein E^{RNS} of pestiviruses of the invention now enables for detectably labeling these pestiviruses. As a result labeled and unlabeled pestiviruses can clearly be distinguished by the absence or presence of RNase activity of the glycoprotein E^{RNS} upon isolation and assaying such enzymatic activity. The determination of presence or absence of this enzymatic activity upon obtaining a sample containing a pestivirus of interest or material thereof can be performed according to standard methods as, for example, described in Example 2 or in Hulst et al. (1994).
Therefore, in a preferred embodiment the present invention relates to a method for distinguishing pestivirus-infected animals from animals vaccinated with a specifically attenuated pestivirus according to the invention, comprising the following steps:
(1) Obtaining a sample from an animal of interest suspected of pestivirus infection or a vaccinated animal;
(2) Determining the absence or presence of RNase activity of a glycoprotein E^{RNS} within said sample;
(3) Correlating the absence of RNase activity of glycoprotein E^{RNS} with a vaccinated animal and correlating the presence of said activity with a pestivirus infection of said animal.

The present invention provides pestiviruses inactivated in their RNase activity residing in glycoprotein E^{RNS} by deletion and/or mutation. Such pestiviruses are easily detected because of the structural consequences resulting from such deletions and/or mutations. The sequence difference of the E^{RNS} gene coding for the altered glycoprotein E^{RNS} is detectable by sequencing techniques. As a result, the present invention provides in a preferred embodiment a method for distinguishing pestivirus-infected animals from animals vaccinated with a specifically attenuated pestivirus according to the invention, comprising the following steps:
(1) Obtaining a sample from an animal of interest suspected of pestivirus infection or a vaccinated animal;
(2) Identifying the nucleotide sequence of a pestivirus genome or protein within said sample;
(3) Correlating the deletions and/or mutations of the E^{RNS} nucleotide sequence as present in the vaccine with a vaccinated animal and correlating the absence of said deletions and/or mutations with a pestivirus infection of said animal.

Furthermore, the structural changes resulting from the altered protein sequence of the glycoprotein E^{RNS} of pestiviruses of the invention can be detected by specific monoclonal or polyclonal antibodies, that do not recognize unaltered proteins. Therefore, in a further embodiment, the present invention relates to a method for distinguishing pestivirus-infected animals from animals vaccinated with an attenuated pestivirus according to the invention, comprising the following steps:
(1) Obtaining a sample from an animal of interest suspected of pestivirus infection or a vaccinated animal;
(2) Identifying a modified E^{RNS} glycoprotein of an attenuated pestivirus by the specific binding of monoclonal or polyclonal antibodies to E^{RNS} glycoproteins present in said sample, said glycoproteins being modified by a method according to the invention, whereby said monoclonal or polyclonal antibodies do not bind to unmodified E^{RNS} glycoproteins;
(3) Correlating the specific binding of said monoclonal or polyclonal antibodies with a vaccinated animal and correlating the absence of antibody binding to a pestivirus infection of said animal under the proviso that the presence of pestiviral material in said animal and/or said sample is established otherwise.

Vice versa, it is also possible to detect the altered and thereby structurally labeled proteins by the absence of binding to specific monoclonal or polyclonal antibodies that recognize unaltered glycoproteins E^{RNS} only, if the presence of pestiviruses can be established otherwise. In a preferred embodiment the present invention relates to a method for distinguishing pestivirus-infected animals from animals vaccinated with an attenuated pestivirus according to the invention, comprising the following steps:
(1) Obtaining a sample from an animal of interest suspected of pestivirus infection or a vaccinated animal;
(2) Identifying an unmodified E^{RNS} glycoprotein of a pestivirus by the specific binding of monoclonal or polyclonal antibodies to E^{RNS} glycoproteins present in said sample, said glycoproteins not being modified by a method according to the invention, whereby said monoclonal or polyclonal antibodies do not bind to modified E^{RNS} glycoproteins;
(3) Correlating the specific binding of said monoclonal or polyclonal antibodies with a pestivirus infection in said animal and correlating the absence of antibody binding to an vaccinated animal under the proviso that the presence of pestiviral material in said animal and/or said sample is established otherwise.

Of course, the structural modification and absence of the RNase activity in the labeled viruses of the invention will result in different immune responses in animals when compared to the responses resulting from unlabeled pestivirus infections. The pestiviruses of the invention elicit a different and distinct immune response, cellular as well as humoral, that differs from unmodified and possibly pathogenic immune responses. For example, glycoproteins E^{RNS} according to the invention will result in polyclonal antibodies that are different in their binding specificity when compared to polyclonal antibodies resulting from unmodified glycoproteins. This difference in binding specificity provides a label for distinguishing animals vaccinated with pestiviruses from the invention from pestivirus field infected animals. Tests for screening sera for specific polyclonal antibodies that either bind to a wildtype epitope or a marker deletion mutation of that epitope for the purpose of differentiating infected and vaccinated animals have been described, for example for pseudorabies-infected and vaccinated pigs (Kit et al., 1991).
In a preferred embodiment the present invention relates to a method for distinguishing pestivirus-infected animals from animals vaccinated with an attenuated pestivirus according to the invention, comprising the following steps:
(1) Obtaining a sample of polyclonal antibodies from an animal of interest suspected of pestivirus infection or a vaccinated animal;
(2) Identifying any specific binding of said polyclonal antibodies to unmodified glycoprotein E^{RNS} or glycoprotein E^{RNS} as modified according to the invention.
(3) Correlating the binding of said polyclonal antibodies to unmodified glycoprotein E^{RNS} with a pestivirus infection and correlating the binding of said polyclonal antibodies to glycoprotein E^{RNS} as modified according to the invention with a vaccinated.

### References

**1. Baker, J.C.** 1987. Bovine viral diarrhea virus: a review. J. Am. Vet. Med.Assoc. **190:** 1449-1458.
**2. Becher, P., König, M., Paton, D.J., Thiel, H.J.,** 1995, Further characterization of border disease virus isolates: evidence for the presence of more than three species within the genus pesivirus. *Virology* 209 (1), 200-206.
**3. Donis, R.O., Corapi, W., and Dubovi, E.J.** 1988. Neutralizing monoclonal antibodies to bovine viral diarrhea virus bind to the 56K to 58K glycoprotein. *J. Gen. Virol.* **69:** 77-86.
**4. Hulst, M.M., Himes, G., Newbigin, E., Moormann, R.J.M.** 1994. Glycoprotein E2 of classical swine fever virus: expression in insect cells and identification as a ribonuclease. *Virology* **200:** 558-565.
**5. Hulst, M.M., F.E. Panoto, A. Hooekmann, H.G.P. van Gennip., and Moormann, R.J.M.** 1998. Inactivation of the RNase activity of glycoprotein E^{rns} of classical swine fever virus results in a cytopathogenic virus. J. *Virol.* **72:** 151-157.
**6. Kit, M. and S. Kit 1991.** Sensitive glycoprotein gill blocking ELISA to distinguish between pseudorabies (Aujeszky's disease) -infected and vaccinated pigs. *Veterinary Microbiology* 28:141-155.
**7. Kunkel, T. A., J. D. Roberts, and R. A. Zakour.** 1987. Rapid and efficient site-specific mutagenesis without phenotypic selection. *Methods Enzymol.* **154**:367-392.
**8. König, Matthias,** 1994, Virus der klassischen Schweinepest: Untersuchungen zur Pathogenese und zur Induktion einer protektiven Immunantwort. Dissertation, Tierärztliche Hochschule Hannover, Germany.
**9. Meyers, G., Rümenapf, T. and Thiel, H.-J.** 1989. Molecular cloning and nucleotide sequence of the genome of hog cholera virus. Virology **171**: 555-567.
**10. Meyers, G., Tautz, N., Becher, P., Thiel, H.-J., & Kümmerer, B.M**. 1996b. Recovery of cytopathogenic and noncytopathogenic bovine viral diarrhea viruses from cDNA constructs. *J. Virol.,* **70**: 8606-8613.
**11. Meyers, G., Thiel, H.-J., and Rümenapf, T.** 1996a. Classical swine fever virus: Recovery of infectious viruses from cDNA constructs and generation of recombinant cytopathogenic swine fever virus. *J. Virol.* **67**:7088-709526.
**12. Moennig, V. and Plagemann, J.** 1992. The pestiviruses. Adv. Virus Res. **41**: 53-91.
**13. Paton, D.J., Lowings, J.P., Barrett, A.D.** 1992. Epitope mapping of the gp53 envelope protein of bovine viral diarrhea virus. *Virology* **190**: 763-772.
**14. Rice, C.M.** 1996. The pestiviruses. In *Fields Virology,* eds. Fields, B.N., Knipe, D.M., & Howley, P.M. (Lippincott-Raven, Philadelphia), pp. 931-959.
**15. Rümenapf, T., Unger, G., Strauss, J.H., and Thiel, H.-J.** 1993. Processing of the evelope glycoproteins of pestiviruses. *J. Virol.* **67**: 3288-3294
**16. Schneider, R., G. Unger, R. Stark, E. Schneider-Scherzer, and H.-J. Thiel.** 1993. Identification of a structural glycoprotein of an RNA virus as a ribonuclease. *Science* **261**: 1169-1171.
**17. Thiel, H.-J., Plagemann, G.W., & Moennig, V.** 1996. The pestiviruses. In *Fields Virology,* eds. Fields, B.N., Knipe, D.M., & Howley, P.M. (Lippincott-Raven, Philadelphia), pp.1059-1073.
**18. Thiel, H.-J., Stark, R., Weiland, E., Rümenapf, T. & Meyers, G.** 1991. Hog cholera virus: molecular composition of virions from a pestivirus. *J. Virol.* **65**: 4705-4712.31.
**19. van Rijn, P.A., van Gennip, H.G., de Meijer, E.J., Moormann, R.J.** 1993. Epitope mapping of envelope glycoprotein E1 of hog cholera virus strain Brescia. *J. Gen. Virol.* **74**: 2053-2060.
**20. Weiland, E., Thiel, H._J., Hess, G., and Weiland, F.** (1989). Development of monoclonal neutralizing antibodies agaist bovine viral diarrhea virus after pretreatment of mice with normal bovine cells and cyclophosphamide. J. Virol. Methods **24**: 237-244.
**21. Weiland, E., Stark, R., Haas, B., Rümenapf, T., Meyers, G. and Thiel, H.-J.** (1990). Pestivirus glycoprotein which induces neutralizing antibodies forms part of a disulfide-linked heterodimer. J. Virology **64**, 3563-3569.
**22. Weiland, E., Ahl, R., Stark, R., Weiland, F. and Thiel, H.-J.** (1992). A second envelope glycoprotein mediates neutralization of a pestivirus, hog cholera virus. J. Virology **66**, 3677-3682.
**23. Windisch, J.M., Schneider, R., Stark, R., Weiland, E., Meyers, G., and Thiel, H.-J.** 1996. RNase of classical swine fever virus: biochemical characterization and inhibition by virus-neutralizing monoclonal antibodies. *J. Virol.* **70**: 352-358

### Examples

### Example 1 Generation of RNase-negative pestivirus mutants

Starting with the full length cDNA clones pA/CSFV (Meyers et al., 1996a) or pA/BVDV (Meyers et al., 1996b), from which infectious cRNA can be obtained by in vitro transcription, subclones were generated. For CSFV, an Xhol/Sspl fragment of pA/CSFV was cloned into pBluescript SK+, cut with Xhol and Smal. For BVDV, an Xhol/Bglll fragment from pA/BVDV was cloned into plasmid pCITE-2C, cut with the same enzymes. Single stranded plasmid DNA was produced from these constructs according to the method of Kunkel (Kunkel et al., 1987) using E. coli CJ 236 cells (BioRad) and the VCMS single strand phage (Stratagene). The single stranded DNA was converted to double strands using the 'Phagemid in vitro Mutagenesis Kit' (BioRad) and the following synthetic oligonucleotides as primers:
- C-297-L:: AGGAGCTTACTTGGGATCTG
- C-346-L:: GGAACAAACTTGGATGGTGT
- C-297-K:: ACAGGAGCTTAAAAGGGATCTGGC
- C-346-K:: ATGGAACAAAAAGGGATGGTGTAA
- C-346-d:: GAATGGAACAAAGGATGGTGTAAC
- B-346-d:: CATGAATGGAACAAAGGTTGGTGCAACTGG

The double stranded plasmid DNA was used for transformation of E.coli XL1-Blue cells (Stratagene). Bacterial colonies harboring plasmids were isolated via ampicillin selection, plasmid DNA was prepared and further analyzed by nucleotide sequencing using the T7 polymerase sequencing kit (Pharmacia). Bacterial clones with plasmids containing the desired mutations and no second site changes were amplified, the plasmid DNA purified and used for construction of full length cDNA clones. In the case of CSFV, an Xhol/Ndel fragment from the mutagenized plasmid was inserted together with an Ndel/Bglll fragment derived from plasmid 578 (pCITE 2A, containing the Xhol/Bglll fragment form pA/CSFV) into pA/CSFV cut with Xhol and Bglll. To obtain the BVDV CP7 mutant, an Xhol/Bglll fragment containing the deletion was inserted into pA/BVDV cut with Xhol and Ncol together with a Bglll/Ncol fragment isolated from pA/BVDV/Ins-. From construct pA/BVDV/Ins- a cRNA can be transcribed that gives rise to a noncytopathogenic BVDV upon transfection in suitable cells (Meyers et al., 1996b).
The different full length clones were amplified, and the plasmids isolated. The presence of the desired mutations was proven by DNA sequencing. After linearization with Srfl (CSFV full length clones) or Smal (BVDV full length clones) cRNA was transcribed as described previously (Meyers et al., 1996ab). RNA was purified by gel filtration and phenol/chloroform extraction, and used for transfection of porcine kidney (PK15) cells or bovine kidney (MDBK clone B2) cells (CSFV or BVDV constructs, respectively). The transfections were analyzed by immunofluorescence with virus specific antisera. In all cases, the desired mutants could be recovered and were amplified by passage on the same cell lines used for the transfection experiments. Further analysis of the CSFV mutants included determination of one step growth curves and characterization of viral RNA by Northern blot with virus specific cDNA probes as well as reverse transcription polymerase chain reaction (RT-PCR) and subsequent sequencing of the PCR fragments to verify the presence of the desired mutations in the viral genome. In all cases the presence of the desired mutation was proven; all of the recovered viruses grew equally well and produced similar amounts of RNA as the virus resulting from the plasmid displaying the wild type sequence.
The viability of the BVDV mutant was shown by transfection of the respective cRNA and splitting of the cells 3 days thereafter. Part of the cells was seeded into a 3.5cm diameter dish, fixed with acetone/methanol at the day thereafter and analysed by immunofluorescence with a mixture of BVDV-specific monoclonal antibodies (Weiland et al., 1989). All cells were found positive whereas a control of cells transfected with noninfectious RNA showed no signal. From a part of the cells transfected with the respective cRNA, an extract was produced by one cycle of freezing and thawing. Fresh cells were infected with this cell extract and proved to be BVDV positive by BVDV specific immunofluorescence 3 days post infection.

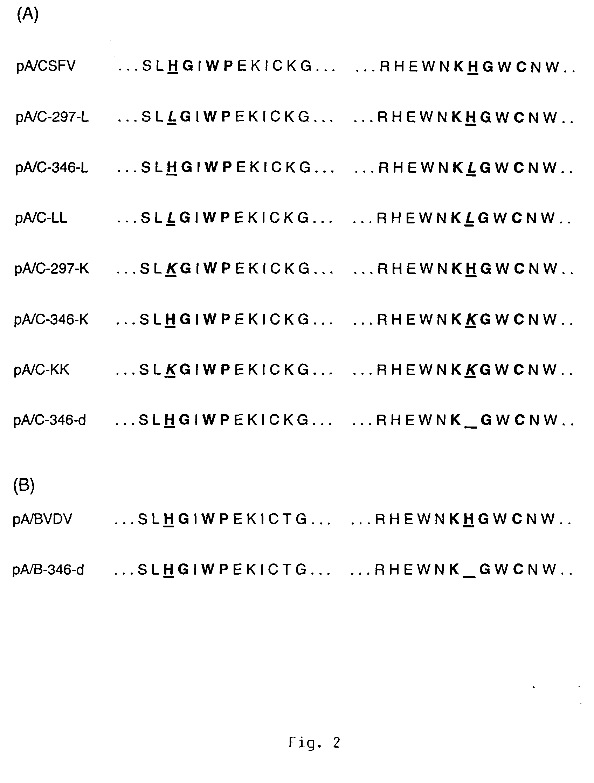

Figure 2 summarizes the different changes introduced into the conserved sequences of E ^{RNS} representing the putative active site of the RNase which are encoded by the indicated virus mutants

### Example 2 Effect of different mutations on RNase activity of E^{RNS}

To check the effect of the different mutations with regard to RNase activity of E^{rns} appropriate cells were infected with the mutant viruses. For CSFV the infection was carried out with a multiplicity of infection (m.o.i.) of 0.01. Infection with wild type virus served as a positive control whereas noninfected cells were used as a negative control. At 48h post infection cells were washed twice with phosphate buffered saline, and lysed in 0.4 ml of lysis buffer (20 mM Tris/HCl; 100 mM NaCl, 1 mM EDTA, 2 mg/ml bovine serum albumin; 1% Triton X 100; 0.1% deoxycholic acid; 0.1% sodium dodecyl sulfate). The lysate was given into 1.5 ml reaction tubes, sonified (Branson sonifier B12, 120 Watt, 20 s in a cup horn water bath), cleared by centrifugation (5 min, 14,000 rpm, Eppendorf Centrifuge, 4°C) and the supernatant subjected to ultracentrifugation (Beckmann table top ultracentifuge, 60 min at 4°C and 45,000 rpm in a TLA 45 rotor). Determination of RNase activity was done in a total volume of 200 µl containing 5 or 50 µl of supernatant of the second centrifugation step and 80 µg of Poly(rU)(Pharmacia) in RNase-assay buffer (40 mM Tris-acetate (pH 6.5), 0.5 mM EDTA, 5 mM dithiothreitol (DTT)). After incubation of the reaction mixture at 37°C for 1 hour 200 µl of 1.2 M perchloric acid, 20 mM LaSO₄ was added. After 15 mm incubation on ice the mixture was centrifugated for 15 min at 4°C and 14,000 rpm in an Eppendorf centrifuge. To the supernatant 3 volumes of water were added and an aliquot of the mixture was analyzed by measuring the optical density at 260 nm using an Ultrospec 3000 spectrophotometer (Pharmacia). In all cases, the mutations introduced into the E^{rns} gene completely abrogated RNase activity (Table 1A).
For the BVDV mutant RNase activity was tested with material obtained after RNA transfection without passage of the recovered viruses. Cells transfected with the appropriate RNA were split 72h post transfection and seeded in two dishes. 24h later, from one dish cell extracts were prepared and analysed for RNase activity as described above. To prove infection, the cells of the second dish were analysed by immunofluorescence with BVDV specific monoclonal antibodies (Weiland et al., 1989) and found 100% positive. Transfection was carried out with RNA transcribed from pA/BVDV/Ins- and from pA/B-346-d, the plasmid derived from pA/BVDV/Ins- by deletion of the codon equivalent to the codon 346 in the CSFV Alfort genome. Nontransfected MDBK cells served as a negative control.

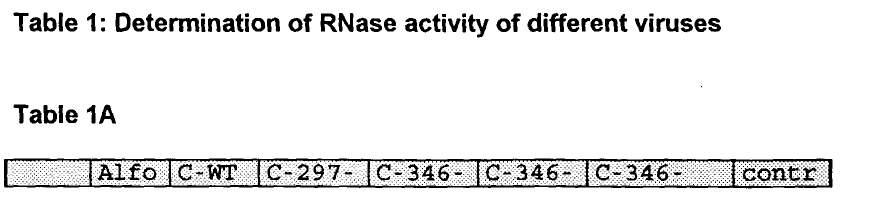

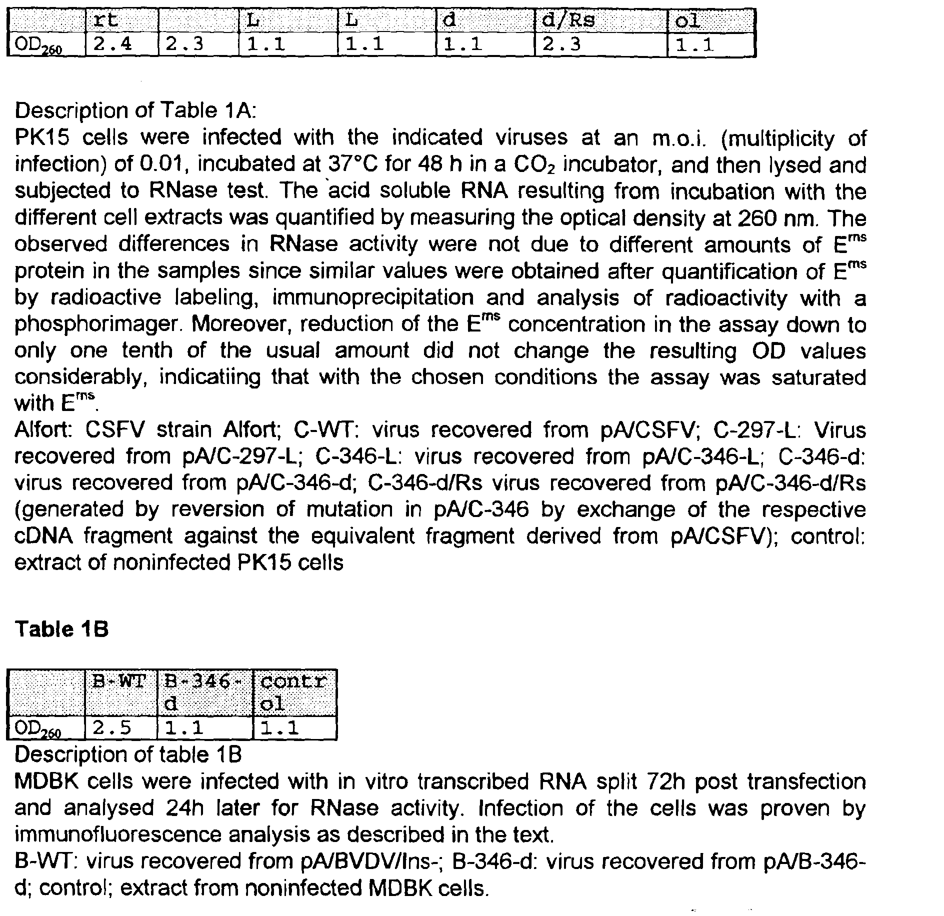

### Example 3 Pathogenicity of CSFV after RNase inactivation

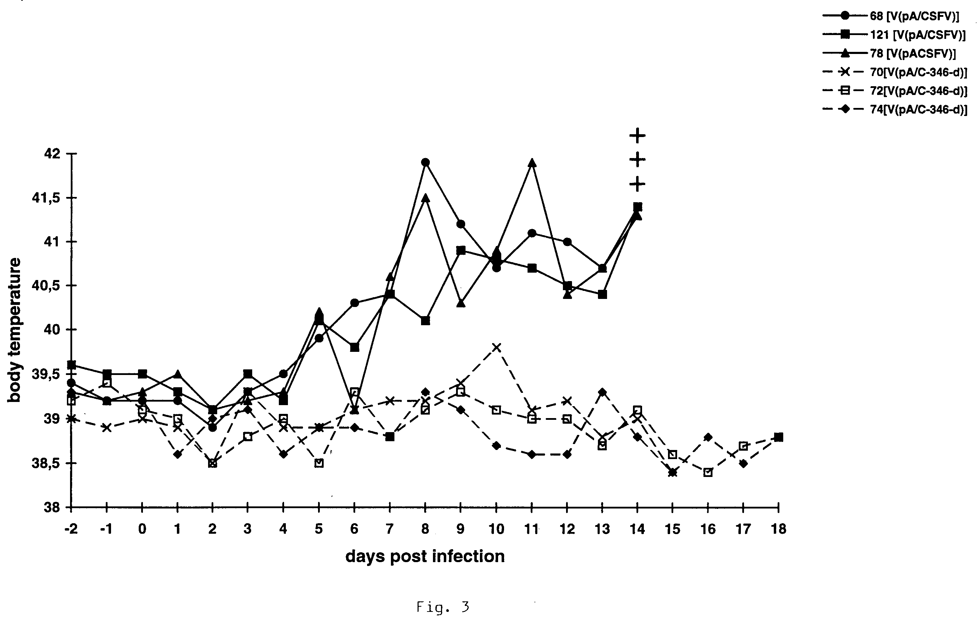

To test, whether the destruction of the RNase activity influences the pathogenicity of pestiviruses in their natural host, animal experiments were conducted with mutant V(pA/C-346-d) (C346-d in tables). Virus recovered from the CSFV full length clone without mutation (V(pA/CSFV)) served as a positive control (C-WT in tables). For each mutant three piglets (breed: German landrace; about 25kg body weight) were used. The infection dose was 1x10⁵ TCID₅₀ per animal; two thirds of the inoculate was administered intranasally (one third in each nostril), one third intramuscularly. The two groups were housed in separate isolation units. Blood was taken from the animals two times before infection and on days 3, 5, 7, 10, 12 and 14. In addition, temperature was recorded daily (Figure 3). The animals infected with the wild type virus showed typical symptoms of classical swine fever like fever) ataxia, anorexia, diarrhea, central nervous disorders, hemorrhages in the skin (Table 2). Virus could be recovered form the blood on days 3 (animal #68) and on days 5, 7, 10, 14 (animals #68, #78, #121) (Table 3) The animals were killed in a moribund stage at day 14 post infection. At this time, no virus neutralizing antibodies could be detected.
In contrast, the animals infected with the mutant did not develop clinical symptoms (Table 2). The temperature stayed normal (Figure 3) over the whole experimental period and the animals never stopped taking up food. At no time virus could be recovered from the blood. Nevertheless, the animals were clearly infected and the virus replicated since all animals developed neutralizing antibodies (Table 4).

**Table 2**

| **Clinical signs after test information:** Animal experiment 1 | | | | | | | | | |
|---|---|---|---|---|---|---|---|---|---|
| Anim. No.: | infected with | clinical signs | | | | | | | |
| | | fever | diarrhea | CNS disorders | anorexia | hemorrhages in skin | apathia | moribund at day of euthanasia | hemorrhages in organs at necropsy |
| #68 | C-WT | + | + | + | + | + | + | + | + |
| #78 | C-WT | + | + | + | + | + | + | + | + |
| #121 | C-WT | + | + | + | + | + | + | + | + |
| #70 | C-346-d | - | - | - | - | - | - | - | n.a. |
| #72 | C-346-d | - | - | - | - | - | - | - | n.a. |
| #74 | C-346-d | - | - | - | - | - | - | - | n.a. |
| Description of Table 2: 6 piglets (German landrace; about 25kg body weight) in two groups (each group were housed separately) were included in the study. 3 animal got infected with CSFV-WT (1·10⁵ TCID₅₀) and 3 animals with C-346-d (1·10⁵ TCID₅₀). Rectal temperature and clinical signs were recorded and summarized as detailed in the table. n.a.: no necropsy was performed. | | | | | | | | | |

**Table 3**

| **Blood cell viremia after test infection** Animal experiment 1 | | | | | | |
|---|---|---|---|---|---|---|
| Animal number | infected with | viremia at days post infection | | | | |
| | | 3 | 5 | 7 | 10 | 14 |
| #68 | C-WT | + | + | + | + | + |
| #78 | C-WT | - | + | + | + | + |
| #121 | C-WT | - | + | + | + | + |
| #70 | C-346-d | - | - | - | - | - |
| #72 | C-346-d | - | - | - | - | - |
| #74 | C-346-d | - | - | - | - | - |
| Description of Table 3: Blood cell viremia was detected by cocultivation of blood with PK15 cells. After incubation at 37°C for 72h cells were washed with PBS, fixed with ice cold acetone/methanol and analyzed for infection by immunofluorescence with a monoclonal antibody specific for glycoprotein E2 (mAb A18, Weiland et al. 1990). | | | | | | |

**Table 4**

| **Development of CSFV specific serum neutralisation titer** | | | | | | | | |
|---|---|---|---|---|---|---|---|---|
| days p.i. | -3 | 0 | 17 | 25 | 69 | 76 | 79 | 87 |
| pig # 70 | - | - | 1:18 | 1:162 | 1:162 | 1:162 | 1:486 | 1:1458 |
| pig # 72 | - | - | 1:18 | 1:54 | 1:486 | 1:1458 | 1:1458 | 1:4374 |
| pig # 74 | - | - | 1:6 | 1:54 | 1:162 | 1:162 | 1:486 | 1:1458 |
| Description of Table 4: Antibody titers of pigs infected with virus mutant C-346-d determined at different time points during the animal experiment 50 µl of the plasma dilution were mixed with 50 µl of medium containing 30 TCID₅₀ of virus (CSFV Alfort/Tübingen). After 90 minutes incubation at 37°C 100 µl of cells (1.5x10⁴ cells) were added and the mixture was seeded in 96 well plates. After 72 h the cells were fixed with ice cold acetone/methanol and analyzed for infection by immunofluorescence with a monoclonal antibody specific for glycoprotein E2 (mAb A18, Weiland et al. 1990). On day 69 post infection the animals were challenged with 2x10⁵ TCID₅₀ of CSFV strain Eystrup. The table gives the highest serum dilution resulting in complete neutralisation of input virus. | | | | | | | | |

### Example 4 Induction of protective immunity by infection with RNase negative virus

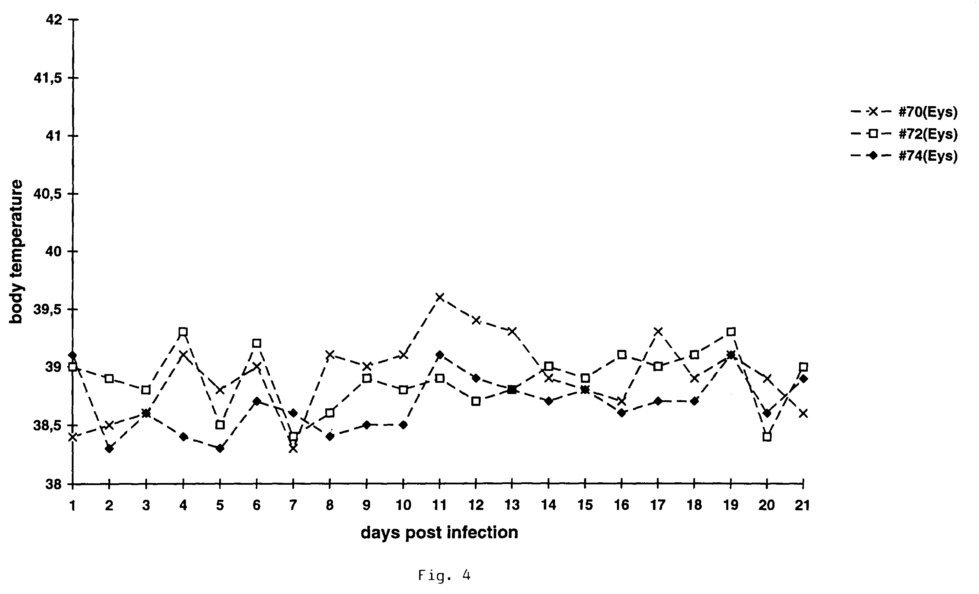

To analyze whether the infection with the mutant virus had led to a protective immunity, a challenge experiment was conducted about 9 weeks after the infection with the CSFV mutant using a highly pathogenic heterologous CSFV strain (strain Eystrup, originated from Behring). 2x10⁵ TCID₅₀ of virus was used for the infection. This amount of virus was found to be sufficient to induce lethal disease in several preceeding experiments (König, 1994). However, the animals previously infected with the CSFV RNase mutant did not show symptoms of disease after challenge infection. Neither fever (Figure 4) nor viremia could be detected but an increase in neutralizing antibodies indicated productive infection and replication of the challenge virus.

### Example 5 Confirmation of attenuation principle

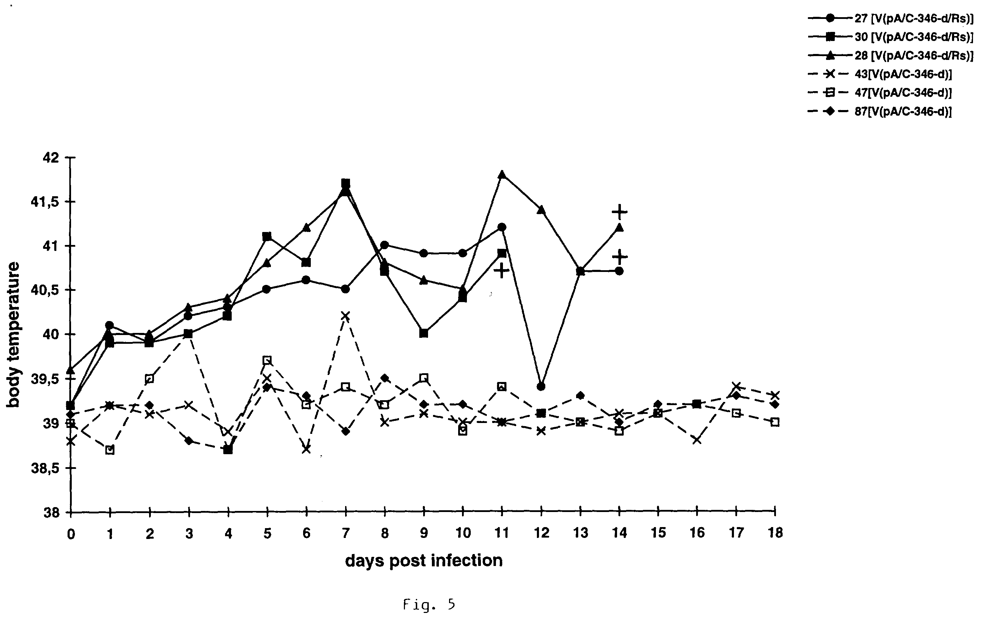

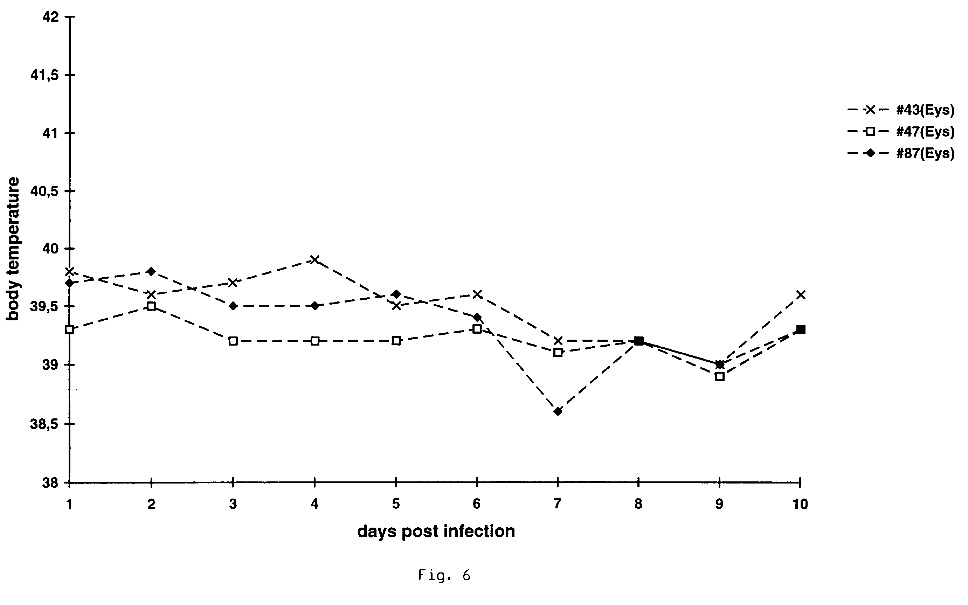

To show, that the observed attenuation of the mutant virus is indeed due to the deletion of the histidine at position 346 of the polyprotein and not a consequence of an unidentified second site mutation, the wild type sequence was restored by exchange of a 1.6 kb Xhol/Ndel fragment of the full length clone pA/C-346-d against the corresponding fragment of pA/CSFV displaying the wild type sequence. The fragment excised from pA/C-346-d was analyzed by nucleotide sequencing for mutations; except for the deletion of the triplet coding for histidine 346 of the polyprotein no difference with regard to the wild type sequence was found. From the cDNA construct with the rescued mutant, virus V(pA/C-346-d/Rs) could be recovered that grew equally well as wild type virus and showed equivalent RNase activity (Table 1A).
In a second animal experiment, the rescued virus was used for infection of pigs. As a control the deletion mutant was used. Again, two groups consisting of three animals were used. As the animals were younger (German landrace, about 20kg) than those in the first experiment 5x10⁴ TCID₅₀ of virus were used for infection this time. Again, the animals infected with the mutant showed no clinical signs (Table 5, Figure 5). Only one animal had fever for one day. Nevertheless, these animals developed neutralising antibodies and were protected against a lethal CSFV challenge. Challenge was again performed by infection with 2x10⁵ TCID₅₀ of challenge strain Eystrup. The animals did not show clinical signs after challenge and the temperature stayed normal (Figure 6). In contrast to the pigs infected with the deletion mutant, the animals inoculated with the rescued wild type virus developed fatal classical swine fever. One animal had to be killed 11 days after infection, the other two 3 days later. All animals showed typical symptoms of classical swine fever, i.e. fever, diarrhea, annorexia, and pathological signs like hemorrhages in different organs including the kidney.

**Table 5**

| **Clinical signs after test infection** Animal experiment 2 | | | | | | | | | |
|---|---|---|---|---|---|---|---|---|---|
| Anim. No.: | infected with | clinical signs | | | | | | | |
| | | fever | diarrhea | CNS disorders | anorexia | hemorrhages in skin | apathia | moribund at day of euthanasia | hemorrhages in organs at necropsy |
| #43 | C-348-d | +* | - | - | - | - | - | - | n.a. |
| #47 | C-346-d | - | - | - | - | - | - | - | n.a. |
| #87 | C-346-d | - | - | - | - | - | - | - | n.a. |
| #27 | C-346-d/RS | + | + | + | + | - | + | + | + |
| #28 | C-346-d/RS | + | + | + | + | - | + | + | + |
| #30 | C-346-d/RS | + | + | + | + | - | + | + | + |
| | | * fever for only 1 day | | | | | | | |
| Table 5: 6 piglets (German landrace; about 20kg body weight) in two groups (each group were housed separately under isolation conditions) were included in the study. 3 animal got infected with mutant C-346-d (5·10⁴ TCID₅₀) and 3 animals with C-346-d/RS (5·10⁴ TCID₅₀). C-346-d/RS was derived from mutant C-346-d by restoring the wild type sequence of E^{RNS} gene. Rectal temperature and clinical signs were recorded and summarized. n.a.: no necropsy was performed. | | | | | | | | | |

### Figure legends

**Figure. 1: The first 495 amino acids as expressed by the Alfort strain of CSFV** The sequence listing shows the first 495 amino acids as expressed by the Alfort strain of CSFV (Meyers et al., 1989). One monomer of the glycoprotein E^{RNS} of said strain corresponds to the amino acids 268 to 494 as described by Rümenapf et at (1993). Residues 295 to 307 and 338 to 357 representing the regions showing homology to plant and fungal RNases (Schneider et al., 1993) are underlined.
**Figure 2: Sequences encoded by the different E**^{**RNS**} **mutants**
   Sequences encoded by the different E^{RNS} mutants derived from the infectious CSFV cDNA clone pA/CSFV (A) or the infectious BVDV cDNA clone pA/BVDV (B) (Meyers at al., 1996 a, b). Only those parts of the E^{RNS} sequence containing the conserved regions with the putative active site residues of the RNase are shown. Residues conserved with regard to known RNases of fungal or plant origin are shown in bold face. Residues 297 and 346 are underlined (numbering based on the polyprotein encoded by the CSFV Alfort genome (Meyers et al., 1989)).
**Figure 3: Rectal temperature curve of animals after test infectio**n
   Daily rectal temperature was recorded from day 2 before till day 18 post infection. Rectal temperature curve is detailed for each animal of the group infected with the virus derived from plasmid pA/CSFV [V(pA/CSFV)] (continuous line) or with the virus derived from plasmid pA/C-346-d [V(pA/C-346-d)] (dotted line).
**Figure 4: Rectal temperature curve of animals after challenge infection**
   Daily rectal temperature was recorded at days 1-21 post challenge virus infection. Animals challenged with a lethal dosis of the CSFV challenge strain Eystrup had been infected with mutant C-346-d [V(pA/C-346-d)] 69 days in before as detailed in the text. Rectal temperature curve is detailed for each animal of the group challenged with 2x10⁵ TCID₅₀ from the CSFV challenge strain Eystrup
**Figure 5: Rectal temperature curve of animals after test infection**
   Daily rectal temperature was recorded at days 0-18 post infection. Rectal temperature curve is detailed for each animal of the two groups infected either with C-346-d [V(pA/C-346-d)] (dotted line) or with the restored virus C-346-d/RS [V(pA/C-346-d/Rs)] (continuous line).
**Figure 6: Rectal temperature after challenge infection animal experiment #2**
   Daily rectal temperature was recorded at days 1-10 post challenge virus infection. Animals challenged with a lethal dose (2x10⁵ TCID₅₀) of the CSFV challenge strain Eystrup had been infected with mutant C-346-d 37 days in before.

## Claims

1. A live vaccine comprising a pestivirus, wherein the RNase activity residing in glycoprotein E^{RNS} is inactivated.

2. The vaccine of claim 1, wherein said RNase activity is inactivated by deletions and/or mutations of at least one amino acid of said glycoprotein.

3. The vaccine according to claim 2, wherein said deletions and/or mutations are located at the amino acids at position 295 to 307 and/or position 338 to 357, as described in figure 1 for the CSFV Alfort strain in an exemplary manner or corresponding thereto in other strains, of said glycoprotein.

4. The vaccine according to any one of claims 1 to 3, wherein said RNase activity is inactivated by deletion or mutation of the amino acid at position 346, as described in figure 1 for the CSFV Alfort strain in an exemplary manner or corresponding thereto in other strains, of said glycoprotein.

5. The vaccine according to any one of claims 1 to 4, wherein said RNase activity is inactivated by the deletion of the histidine residue at position 346, as described in figure 1 for the CSFV Alfort strain in an exemplary manner or corresponding thereto in other strains, of said glycoprotein.

6. A pestivirus, wherein the RNase activity residing in glycoprotein E^{RNS} is inactivated by deletions and/or mutations of at least one amino acid of said glycoprotein with the proviso that the amino acids at position 297 and/or 346, as described in figure 1 for the CSFV Alfort strain in an exemplary manner or corresponding thereto in other strains, of said glycoprotein are not lysine.

7. The pestivirus of claim 6, wherein said RNase activity is inactivated by deletions and/or mutations located at the amino acids at position 295 to 307 and/or position 338 to 357, as described in figure 1 for the CSFV Alfort strain in an exemplary manner or corresponding thereto in other strains, of said glycoprotein.

8. The pestivirus of claim 6 or 7, wherein said RNase activity is inactivated by deletion or mutation of the amino acid at position 346, as described in figure 1 for the CSFV Alfort strain in an exemplary manner or corresponding thereto in other strains, of said glycoprotein.

9. The pestivirus according to any one of claims 6 to 8, wherein said RNase activity is inactivated by the deletion of the histidine residue at position 346, as described in figure 1 for the CSFV Alfort strain in an exemplary manner or corresponding thereto in other strains, of said glycoprotein.

10. A nucleic acid coding for a glycoprotein E^{RNS}, wherein the RNase activity residing in said glycoprotein is inactivated by deletions and/or mutations of at least one amino acid of said glycoprotein with the proviso that the amino acids at position 297 and/or 346, as described in figure 1 for the CSFV Alfort strain in an exemplary manner or corresponding thereto in other strains, of said glycoprotein are not lysine.

11. The nucleic acid of claim 10, wherein said RNase activity is inactivated by deletions and/or mutations that are located at the amino acids at position 295 to 307 and/or position 338 to 357, as described in figure 1 for the CSFV Alfort strain in an exemplary manner or corresponding thereto in other strains, of said glycoprotein.

12. The nucleic acid of claim 10 or 11, wherein said RNase activity is inactivated by deletion or mutation of the amino acid at position 346, as described in figure 1 for the CSFV Alfort strain in an exemplary manner or corresponding thereto in other strains, of said glycoprotein.

13. The nucleic acid according to any one of claims 10 to 12, wherein said RNase activity is inactivated by the deletion of the histidine residue at position 346, as described in figure 1 for the CSFV Alfort strain in an exemplary manner or corresponding thereto in other strains, of said glycoprotein.

14. A pharmaceutical composition comprising a vaccine according to any one of claims 1 to 5, and/or a pestivirus according to any one of claims 6 to 9, and/or a nucleotide sequence according to any one of claims 10 to 13.

15. A method for attenuating pestiviruses characterized in that the RNase activity residing in glycoprotein E^{RNS} is inactivated.

16. The method of claim 15, wherein said RNase activity is inactivated by deletions and/or mutations of at least one amino acid of said glycoprotein.

17. The method of claim 15 or 16, wherein said deletions and/or mutations are located at the amino acids at position 295 to 307 and/or position 338 to 357, as described in figure 1 for the CSFV Alfort strain in an exemplary manner or corresponding thereto in other strains, of said glycoprotein.

18. The method according to any one of claims 15 to 17, wherein said RNase activity is inactivated by deletion or mutation of the amino acid at position 346, as described in figure 1 for the CSFV Alfort strain in an exemplary manner or corresponding thereto in other strains, of said glycoprotein.

19. The method according to any one of claims 15 to 18, wherein said RNase activity is inactivated by the deletion of the histidine residue at position 346, as described in figure 1 for the CSFV Alfort strain in an exemplary manner or corresponding thereto in other strains, of said glycoprotein.

20. A method for producing a specifically attenuated vaccine characterized in that the RNase activity residing in glycoprotein E^{RNS} is inactivated.

21. The method of claim 20, wherein said RNase activity is inactivated by deletions and/or mutations of at least one amino acid of said glycoprotein.

22. The method of claim 20 or 21, wherein said deletions and/or mutations are located at the amino acids at position 295 to 307 and/or position 338 to 357, as described in figure 1 for the CSFV Alfort strain in an exemplary manner or corresponding thereto in other strains, of said glycoprotein.

23. The method according to any one of claims 20 to 22, wherein said RNase activity is inactivated by deletion or mutation of the amino acid at position 346, as described in figure 1 for the CSFV Alfort strain in an exemplary manner or corresponding thereto in other strains, of said glycoprotein.

24. The method according to any one of claims 20 to 23, wherein said RNase activity is inactivated by the deletion of the histidine residue at position 346, as described in figure 1 for the CSFV Alfort strain in an exemplary manner or corresponding thereto in other strains, of said glycoprotein.

25. A method for detectably labeling pestiviruses characterized in that the RNase activity residing in glycoprotein E^{RNS} is inactivated.

26. The method of claim 25, wherein said RNase activity is inactivated by deletions and/or mutations of at least one amino acid of said glycoprotein.

27. The method of claim 25 or 26, wherein said deletions and/or mutations are located at the amino acids at position 295 to 307 and/or position 338 to 357, as described in figure 1 for the CSFV Alfort strain in an exemplary manner or corresponding thereto in other strains, of said glycoprotein.

28. The method according to any one of claims 25 to 27, wherein said RNase activity is inactivated by deletion or mutation of the amino acid at position 346, as described in figure 1 for the CSFV Alfort strain in an exemplary manner or corresponding thereto in other strains, of said glycoprotein.

29. The method according to any one of claims 25 to 28, wherein said RNase activity is inactivated by the deletion of the histidine residue at position 346, as described in figure 1 for the CSFV Alfort strain in an exemplary manner or corresponding thereto in other strains, of said glycoprotein.

30. A method for the prophylaxis and treatment of pestivirus infections in animals characterized in that a vaccine according to any one of claims 1 to 5 or a pharmaceutical composition according to claim 14 is applied to an animal in need of such prophylaxis or treatment.

31. A process for the preparation of specifically attenuated pestiviruses characterized in that the RNase activity residing in glycoprotein E^{RNS} is inactivated.

32. The process according to claim 31, wherein said RNase activity is inactivated by deletions and/or mutations of at least one amino acid of said glycoprotein.

33. The process according to claim 31 or 32, wherein said deletions and/or mutations are located at the amino acids at position 295 to 307 and/or position 338 to 357, as described in figure 1 for the CSFV Alfort strain in an exemplary manner or corresponding thereto in other strains, of said glycoprotein.

34. The process according to any one of claims 31 to 33, wherein said RNase activity is inactivated by deletion or mutation of the amino acid at position 346, as described in figure 1 for the CSFV Alfort strain in an exemplary manner or corresponding thereto in other strains, of said glycoprotein.

35. The process according to any one of claims 31 to 34, wherein said RNase activity is inactivated by the deletion of the histidine residue at position 346, as described in figure 1 for the CSFV Alfort strain in an exemplary manner or corresponding thereto in other strains, of said glycoprotein.

36. A process for the preparation of specifically labeled pestiviruses characterized in that the RNase activity residing in glycoprotein E^{RNS} is inactivated.

37. The process according to claim 36, wherein said RNase activity is inactivated by deletions and/or mutations of at least one amino acid of said glycoprotein.

38. The process according to claim 36 or 37, wherein said deletions and/or mutations are located at the amino acids at position 295 to 307 and/or position 338 to 357, as described in figure 1 for the CSFV Alfort strain in an exemplary manner or corresponding thereto in other strains, of said glycoprotein.

39. The process according to any one of claims 36 to 38, wherein said RNase activity is inactivated by deletion or mutation of the amino acid at position 346, as described in figure 1 for the CSFV Alfort strain in an exemplary manner or corresponding thereto in other strains, of said glycoprotein.

40. The process according to any one of claims 36 to 39, wherein said RNase activity is inactivated by the deletion of the histidine residue at position 346, as described in figure 1 for the CSFV Alfort strain in an exemplary manner or corresponding thereto in other strains, of said glycoprotein.

41. Use of a vaccine of any one of claims 1 to 5 for the prophylaxis and treatment of pestivirus infections in animals.

42. Use of a pharmaceutical composition of claim 14 for the prophylaxis and treatment of pestivirus infections in animals.

43. Use of a pestivirus of any one of claims 6 to 9 and/or a nucleotide sequence according to any one of claims 10 to 13 for the preparation of a vaccine or a pharmaceutical composition.

44. A method for distinguishing pestivirus-infected animals from animals vaccinated with a specifically attenuated pestivirus, wherein said specifically attenuated pestivirus is attenuated according to a method of any one of claims 15 to 19, comprising the following steps:
(1) Obtaining a sample from an animal of interest suspected of pestivirus infection or a vaccinated animal;
(2) Identifying the nucleotide sequence of a pestivirus within said sample;
(3) Correlating the deletions and/or mutations of the E^{RNS} nucleotide sequence as present in the vaccine with a vaccinated animal and correlating the absence of said deletions and/or mutations with a pestivirus infection of said animal.

45. The method of claim 44, comprising the following steps:
(1) Obtaining a sample from an animal of interest suspected of pestivirus infection or a vaccinated animal;
(2) Identifying a modified E^{RNS} glycoprotein of an attenuated pestivirus by the specific binding of monoclonal or polyclonal antibodies to E^{RNS} glycoproteins present in said sample, said glycoproteins being modified by a method according to any one of claims 15 to 19, whereby said monoclonal or polyclonal antibodies do not bind to unmodified E^{RNS} glycoproteins;
(4) Correlating the specific binding of said monoclonal or polyclonal antibodies with a vaccinated animal and correlating the absence of antibody binding to a pestivirus infection of said animal under the proviso that the presence of pestiviral material in said animal and/or said sample is established otherwise.

46. The method of claim 44, comprising the following steps:
(1) Obtaining a sample from an animal of interest suspected of pestivirus infection or a vaccinated animal;
(2) Identifying an unmodified E^{RNS} glycoprotein of a pestivirus by the specific binding of monoclonal or polyclonal antibodies to E^{RNS} glycoproteins present in said sample, said glycoproteins not being modified by a method according to any one of claims 15 to 19, whereby said monoclonal or polyclonal antibodies do not bind to modified E^{RNS} glycoproteins;
(3) Correlating the specific binding of said monoclonal or polyclonal antibodies with a pestivirus infection in said animal and correlating the absence of antibody binding to an vaccinated animal under the proviso that the presence of pestiviral material in said animal and/or said sample is established otherwise.

47. The method of claim 44, comprising the following steps:
(1) Obtaining a sample from an animal of interest suspected of pestivirus infection or a vaccinated animal;
(2) Determining the absence or presence of RNase activity of a glycoprotein E^{RNS} within said sample;
(3) Correlating the absence of RNase activity of glycoprotein E^{RNS} with a vaccinated animal and correlating the presence of said activity with a pestivirus infection of said animal.

48. The method of claim 44, comprising the following steps:
(1) Obtaining a sample of polyclonal antibodies from an animal of interest suspected of pestivirus infection or a vaccinated animal;
(2) Identifying any specific binding of said polyclonal antibodies to unmodified glycoprotein E^{RNS} or glycoprotein E^{RNS} as modified according to the invention.
(3) Correlating the binding of said polyclonal antibodies to unmodified glycoprotein E^{RNS} with a pestivirus infection and correlating the binding of said polyclonal antibodies to glycoprotein E^{RNS} as modified according to the invention with a vaccinated.


SEQUENCE LISTING

(1) GENERAL INFORMATION:

(i) APPLICANT:
(A) NAME: Boehringer Ingelheim Vetmedica GmbH
(B) STREET: Binger Strasse 173
(C) CITY: Ingelheim
(E) COUNTRY: Germany
(F) POSTAL CODE (ZIP): 55216
(G) TELEPHONE: 06132777065
(H) TELEFAX: 06132774377

(ii) TITLE OF INVENTION: Attenuated pestiviruses

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98110356.7

(2) INFORMATION FOR SEQ ID NO: 1:

(i) SEQUENCE CHARACTERISTICS:
(A) LENGTH: 495 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: internal

(vi) ORIGINAL SOURCE:
(A) ORGANISM: Pestivirus Glycoprotein Erns
(B) STRAIN: CSFV Classical Swine Fever Virus
(C) INDIVIDUAL ISOLATE: Alfort

(xi) SEQUENCE DESCRIPTION: SEQ ID NO: 1:

Met Glu Leu Asn His Phe Glu Leu Leu Tyr Lys Thr Ser Lys Gln Lys
1               5                   10                  15

Pro Val Gly Val Glu Glu Pro Val Tyr Asp Thr Ala Gly Arg Pro Leu
            20                  25                  30

Phe Gly Asn Pro Ser Glu Val His Pro Gln Ser Thr Leu Lys Leu Pro
        35                  40                  45

His Asp Arg Gly Arg Gly Asp Ile Arg Thr Thr Leu Arg Asp Leu Pro
    50                  55                  60

Arg Lys Gly Asp Cys Arg Ser Gly Asn His Leu Gly Pro Val Ser Gly
65                  70                  75                  80

Ile Tyr Ile Lys Pro Gly Pro Val Tyr Tyr Gln Asp Tyr Thr Gly Pro
                85                  90                  95

Val Tyr His Arg Ala Pro Leu Glu Phe Phe Asp Glu Ala Gln Phe Cys
            100                 105                 110

Glu Val Thr Lys Arg Ile Gly Arg Val Thr Gly Ser Asp Gly Lys Leu
        115                 120                 125

Tyr His Ile Tyr Val Cys Val Asp Gly Cys Ile Leu Leu Lys Leu Ala
    130                 135                 140

Lys Arg Gly Thr Pro Arg Thr Leu Lys Trp Ile Arg Asn Phe Thr Asn
145                 150                 155                 160

Cys Pro Leu Trp Val Thr Ser Cys Ser Asp Asp Gly Ala Ser Gly Ser
                165                 170                 175

Lys Asp Lys Lys Pro Asp Arg Met Asn Lys Gly Lys Leu Lys Ile Ala
            180                 185                 190

Pro Arg Glu His Glu Lys Asp Ser Lys Thr Lys Pro Pro Asp Ala Thr
        195                 200                 205

Ile Val Val Glu Gly Val Lys Tyr Gln Ile Lys Lys Lys Gly Lys Val
    210                 215                 220

Lys Gly Lys Asn Thr Gln Asp Gly Leu Tyr His Asn Lys Asn Lys Pro
225                 230                 235                 240

Pro Glu Ser Arg Lys Lys Leu Glu Lys Ala Leu Leu Ala Trp Ala Val
                245                 250                 255

Ile Thr Ile Leu Leu Tyr Gln Pro Val Ala Ala Glu Asn Ile Thr Gln
            260                 265                 270

Trp Asn Leu Ser Asp Asn Gly Thr Asn Gly Ile Gln Arg Ala Met Tyr
        275                 280                 285

Leu Arg Gly Val Asn Arg Ser Leu His Gly Ile Trp Pro Glu Lys Ile
    290                 295                 300

Cys Lys Gly Val Pro Thr His Leu Ala Thr Asp Thr Glu Leu Lys Glu
305                 310                 315                 320

Ile Arg Gly Met Met Asp Ala Ser Glu Arg Thr Asn Tyr Thr Cys Cys
                325                 330                 335

Arg Leu Gln Arg His Glu Trp Asn Lys His Gly Trp Cys Asn Trp Tyr
            340                 345                 350

Asn Ile Asp Pro Trp Ile Gln Leu Met Asn Arg Thr Gln Thr Asn Leu
        355                 360                 365

Thr Glu Gly Pro Pro Asp Lys Glu Cys Ala Val Thr Cys Arg Tyr Asp
    370                 375                 380

Lys Asn Thr Asp Val Asn Val Val Thr Gln Ala Arg Asn Arg Pro Thr
385                 390                 395                 400

Thr Leu Thr Gly Cys Lys Lys Gly Lys Asn Phe Ser Phe Ala Gly Thr
                405                 410                 415

Val Ile Glu Gly Pro Cys Asn Phe Asn Val Ser Val Glu Asp Ile Leu
            420                 425                 430

Tyr Gly Asp His Glu Cys Gly Ser Leu Leu Gln Asp Thr Ala Leu Tyr
        435                 440                 445

Leu Leu Asp Gly Met Thr Asn Thr Ile Glu Asn Ala Arg Gln Gly Ala
    450                 455                 460

Ala Arg Val Thr Ser Trp Leu Gly Arg Gln Leu Ser Thr Ala Gly Lys
465                 470                 475                 480

Lys Leu Glu Arg Arg Ser Lys Thr Trp Phe Gly Ala Tyr Ala Leu
                485                 490                 495

SEQUENCE LISTING

(1) GENERAL INFORMATION:

(i) APPLICANT:
(A) NAME: Boehringer Ingelheim Vetmedica GmbH
(B) STREET: Binger Strasse 173
(C) CITY: Ingelheim
(E) COUNTRY: Germany
(F) POSTAL CODE (ZIP): 55216
(G) TELEPHONE: 06132777065
(H) TELEFAX: 06132774377

(ii) TITLE OF INVENTION: Attenuated pestiviruses

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98110356.7

(2) INFORMATION FOR SEQ ID NO: 1:

(i) SEQUENCE CHARACTERISTICS:
(A) LENGTH: 495 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: internal

(vi) ORIGINAL SOURCE:
(A) ORGANISM: Pestivirus Glycoprotein Erns-297-L
(B) STRAIN: CSFV Classical Swine Fever Virus
(C) INDIVIDUAL ISOLATE: Alfort

(xi) SEQUENCE DESCRIPTION: SEQ ID NO: 1:

Met Glu Leu Asn His Phe Glu Leu Leu Tyr Lys Thr Ser Lys Gln Lys
1               5                   10                  15

Pro Val Gly Val Glu Glu Pro Val Tyr Asp Thr Ala Gly Arg Pro Leu
            20                  25                  30

Phe Gly Asn Pro Ser Glu Val His Pro Gln Ser Thr Leu Lys Leu Pro
        35                  40                  45

His Asp Arg Gly Arg Gly Asp Ile Arg Thr Thr Leu Arg Asp Leu Pro
    50                  55                  60

Arg Lys Gly Asp Cys Arg Ser Gly Asn His Leu Gly Pro Val Ser Gly
65                  70                  75                  80

Ile Tyr Ile Lys Pro Gly Pro Val Tyr Tyr Gln Asp Tyr Thr Gly Pro
                85                  90                  95

Val Tyr His Arg Ala Pro Leu Glu Phe Phe Asp Glu Ala Gln Phe Cys
            100                 105                 110

Glu Val Thr Lys Arg Ile Gly Arg Val Thr Gly Ser Asp Gly Lys Leu
        115                 120                 125

Tyr His Ile Tyr Val Cys Val Asp Gly Cys Ile Leu Leu Lys Leu Ala
    130                 135                 140

Lys Arg Gly Thr Pro Arg Thr Leu Lys Trp Ile Arg Asn Phe Thr Asn
145                 150                 155                 160

Cys Pro Leu Trp Val Thr Ser Cys Ser Asp Asp Gly Ala Ser Gly Ser
                165                 170                 175

Lys Asp Lys Lys Pro Asp Arg Met Asn Lys Gly Lys Leu Lys Ile Ala
            180                 185                 190

Pro Arg Glu His Glu Lys Asp Ser Lys Thr Lys Pro Pro Asp Ala Thr
        195                 200                 205

Ile Val Val Glu Gly Val Lys Tyr Gln Ile Lys Lys Lys Gly Lys Val
    210                 215                 220

Lys Gly Lys Asn Thr Gln Asp Gly Leu Tyr His Asn Lys Asn Lys Pro
225                 230                 235                 240

Pro Glu Ser Arg Lys Lys Leu Glu Lys Ala Leu Leu Ala Trp Ala Val
                245                 250                 255

Ile Thr Ile Leu Leu Tyr Gln Pro Val Ala Ala Glu Asn Ile Thr Gln
            260                 265                 270

Trp Asn Leu Ser Asp Asn Gly Thr Asn Gly Ile Gln Arg Ala Met Tyr
        275                 280                 285

Leu Arg Gly Val Asn Arg Ser Leu Leu Gly Ile Trp Pro Glu Lys Ile
    290                 295                 300

Cys Lys Gly Val Pro Thr His Leu Ala Thr Asp Thr Glu Leu Lys Glu
305                 310                 315                 320

Ile Arg Gly Met Met Asp Ala Ser Glu Arg Thr Asn Tyr Thr Cys Cys
                325                 330                 335

Arg Leu Gln Arg His Glu Trp Asn Lys His Gly Trp Cys Asn Trp Tyr
            340                 345                 350

Asn Ile Asp Pro Trp Ile Gln Leu Met Asn Arg Thr Gln Thr Asn Leu
        355                 360                 365

Thr Glu Gly Pro Pro Asp Lys Glu Cys Ala Val Thr Cys Arg Tyr Asp
    370                 375                 380

Lys Asn Thr Asp Val Asn Val Val Thr Gln Ala Arg Asn Arg Pro Thr
385                 390                 395                 400

Thr Leu Thr Gly Cys Lys Lys Gly Lys Asn Phe Ser Phe Ala Gly Thr
                405                 410                 415

Val Ile Glu Gly Pro Cys Asn Phe Asn Val Ser Val Glu Asp Ile Leu
            420                 425                 430

Tyr Gly Asp His Glu Cys Gly Ser Leu Leu Gln Asp Thr Ala Leu Tyr
        435                 440                 445

Leu Leu Asp Gly Met Thr Asn Thr Ile Glu Asn Ala Arg Gln Gly Ala
    450                 455                 460

Ala Arg Val Thr Ser Trp Leu Gly Arg Gln Leu Ser Thr Ala Gly Lys
465                 470                 475                 480

Lys Leu Glu Arg Arg Ser Lys Thr Trp Phe Gly Ala Tyr Ala Leu
                485                 490                 495

SEQUENCE LISTING

(1) GENERAL INFORMATION:

(i) APPLICANT:
(A) NAME: Boehringer Ingelheim Vetmedica GmbH
(B) STREET: Binger Strasse 173
(C) CITY: Ingelheim
(E) COUNTRY: Germany
(F) POSTAL CODE (ZIP): 55216
(G) TELEPHONE: 06132777065
(H) TELEFAX: 061324377

(ii) TITLE OF INVENTION: Attenuated pestiviruses

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98110356.7

(2) INFORMATION FOR SEQ ID NO: 1:

(i) SEQUENCE CHARACTERISTICS:
(A) LENGTH: 495 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: internal

(vi) ORIGINAL SOURCE:
(A) ORGANISM: Pestivirus Glycoprotein Erns
(B) STRAIN: CSFV Classical Swine Fever Virus -346-L
(C) INDIVIDUAL ISOLATE: Alfort

(xi) SEQUENCE DESCRIPTION: SEQ ID NO: 1:

Met Glu Leu Asn His Phe Glu Leu Leu Tyr Lys Thr Ser Lys Gln Lys
1               5                   10                  15

Pro Val Gly Val Glu Glu Pro Val Tyr Asp Thr Ala Gly Arg Pro Leu
            20                  25                  30

Phe Gly Asn Pro Ser Glu Val His Pro Gln Ser Thr Leu Lys Leu Pro
        35                  40                  45

His Asp Arg Gly Arg Gly Asp Ile Arg Thr Thr Leu Arg Asp Leu Pro
    50                  55                  60

Arg Lys Gly Asp Cys Arg Ser Gly Asn His Leu Gly Pro Val Ser Gly
65                  70                  75                  80

Ile Tyr Ile Lys Pro Gly Pro Val Tyr Tyr Gln Asp Tyr Thr Gly Pro
                85                  90                  95

Val Tyr His Arg Ala Pro Leu Glu Phe Phe Asp Glu Ala Gln Phe Cys
            100                 105                 110

Glu Val Thr Lys Arg Ile Gly Arg Val Thr Gly Ser Asp Gly Lys Leu
        115                 120                 125

Tyr His Ile Tyr Val Cys Val Asp Gly Cys Ile Leu Leu Lys Leu Ala
    130                 135                 140

Lys Arg Gly Thr Pro Arg Thr Leu Lys Trp Ile Arg Asn Phe Thr Asn
145                 150                 155                 160

Cys Pro Leu Trp Val Thr Ser Cys Ser Asp Asp Gly Ala Ser Gly Ser
                165                 170                 175

Lys Asp Lys Lys Pro Asp Arg Met Asn Lys Gly Lys Leu Lys Ile Ala
            180                 185                 190

Pro Arg Glu His Glu Lys Asp Ser Lys Thr Lys Pro Pro Asp Ala Thr
        195                 200                 205

Ile Val Val Glu Gly Val Lys Tyr Gln Ile Lys Lys Lys Gly Lys Val
    210                 215                 220

Lys Gly Lys Asn Thr Gln Asp Gly Leu Tyr His Asn Lys Asn Lys Pro
225                 230                 235                 240

Pro Glu Ser Arg Lys Lys Leu Glu Lys Ala Leu Leu Ala Trp Ala Val
                245                 250                 255

Ile Thr Ile Leu Leu Tyr Gln Pro Val Ala Ala Glu Asn Ile Thr Gln
            260                 265                 270

Trp Asn Leu Ser Asp Asn Gly Thr Asn Gly Ile Gln Arg Ala Met Tyr
        275                 280                 285

Leu Arg Gly Val Asn Arg Ser Leu His Gly Ile Trp Pro Glu Lys Ile
    290                 295                 300

Cys Lys Gly Val Pro Thr His Leu Ala Thr Asp Thr Glu Leu Lys Glu
305                 310                 315                 320

Ile Arg Gly Met Met Asp Ala Ser Glu Arg Thr Asn Tyr Thr Cys Cys
                325                 330                 335

Arg Leu Gln Arg His Glu Trp Asn Lys Leu Gly Trp Cys Asn Trp Tyr
            340                 345                 350

Asn Ile Asp Pro Trp Ile Gln Leu Met Asn Arg Thr Gln Thr Asn Leu
        355                 360                 365

Thr Glu Gly Pro Pro Asp Lys Glu Cys Ala Val Thr Cys Arg Tyr Asp
    370                 375                 380

Lys Asn Thr Asp Val Asn Val Val Thr Gln Ala Arg Asn Arg Pro Thr
385                 390                 395                 400

Thr Leu Thr Gly Cys Lys Lys Gly Lys Asn Phe Ser Phe Ala Gly Thr
                405                 410                 415

Val Ile Glu Gly Pro Cys Asn Phe Asn Val Ser Val Glu Asp Ile Leu
            420                 425                 430

Tyr Gly Asp His Glu Cys Gly Ser Leu Leu Gln Asp Thr Ala Leu Tyr
        435                 440                 445

Leu Leu Asp Gly Met Thr Asn Thr Ile Glu Asn Ala Arg Gln Gly Ala
    450                 455                 460

Ala Arg Val Thr Ser Trp Leu Gly Arg Gln Leu Ser Thr Ala Gly Lys
465                 470                 475                 480

Lys Leu Glu Arg Arg Ser Lys Thr Trp Phe Gly Ala Tyr Ala Leu
                485                 490                 495

SEQUENCE LISTING

(1) GENERAL INFORMATION:

(i) APPLICANT:
(A) NAME: Boehringer Ingelheim Vetmedica GmbH
(B) STREET: Binger Strasse 173
(C) CITY: Ingelheim
(E) COUNTRY: Germany
(F) POSTAL CODE (ZIP): 55216
(G) TELEPHONE: 06132777065
(H) TELEFAX: 06132774377

(ii) TITLE OF INVENTION: Attenuated pestiviruses

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98110356.7

(2) INFORMATION FOR SEQ ID NO: 1:

(i) SEQUENCE CHARACTERISTICS:
(A) LENGTH: 495 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: internal

(vi) ORIGINAL SOURCE:
(A) ORGANISM: Pestivirus Glycoprotein Erns-297-L-346-L
(B) STRAIN: CSFV Classical Swine Fever Virus
(C) INDIVIDUAL ISOLATE: Alfort

(xi) SEQUENCE DESCRIPTION: SEQ ID NO: 1:

Met Glu Leu Asn His Phe Glu Leu Leu Tyr Lys Thr Ser Lys Gln Lys
1               5                   10                  15

Pro Val Gly Val Glu Glu Pro Val Tyr Asp Thr Ala Gly Arg Pro Leu
            20                  25                  30

Phe Gly Asn Pro Ser Glu Val His Pro Gln Ser Thr Leu Lys Leu Pro
        35                  40                  45

His Asp Arg Gly Arg Gly Asp Ile Arg Thr Thr Leu Arg Asp Leu Pro
    50                  55                  60

Arg Lys Gly Asp Cys Arg Ser Gly Asn His Leu Gly Pro Val Ser Gly
65                  70                  75                  80

Ile Tyr Ile Lys Pro Gly Pro Val Tyr Tyr Gln Asp Tyr Thr Gly Pro
                85                  90                  95

Val Tyr His Arg Ala Pro Leu Glu Phe Phe Asp Glu Ala Gln Phe Cys
            100                 105                 110

Glu Val Thr Lys Arg Ile Gly Arg Val Thr Gly Ser Asp Gly Lys Leu
        115                 120                 125

Tyr His Ile Tyr Val Cys Val Asp Gly Cys Ile Leu Leu Lys Leu Ala
    130                 135                 140

Lys Arg Gly Thr Pro Arg Thr Leu Lys Trp Ile Arg Asn Phe Thr Asn
145                 150                 155                 160

Cys Pro Leu Trp Val Thr Ser Cys Ser Asp Asp Gly Ala Ser Gly Ser
                165                 170                 175

Lys Asp Lys Lys Pro Asp Arg Met Asn Lys Gly Lys Leu Lys Ile Ala
            180                 185                 190

Pro Arg Glu His Glu Lys Asp Ser Lys Thr Lys Pro Pro Asp Ala Thr
        195                 200                 205

Ile Val Val Glu Gly Val Lys Tyr Gln Ile Lys Lys Lys Gly Lys Val
    210                 215                 220

Lys Gly Lys Asn Thr Gln Asp Gly Leu Tyr His Asn Lys Asn Lys Pro
225                 230                 235                 240

Pro Glu Ser Arg Lys Lys Leu Glu Lys Ala Leu Leu Ala Trp Ala Val
                245                 250                 255

Ile Thr Ile Leu Leu Tyr Gln Pro Val Ala Ala Glu Asn Ile Thr Gln
            260                 265                 270

Trp Asn Leu Ser Asp Asn Gly Thr Asn Gly Ile Gln Arg Ala Met Tyr
        275                 280                 285

Leu Arg Gly Val Asn Arg Ser Leu Leu Gly Ile Trp Pro Glu Lys Ile
    290                 295                 300

Cys Lys Gly Val Pro Thr His Leu Ala Thr Asp Thr Glu Leu Lys Glu
305                 310                 315                 320

Ile Arg Gly Met Met Asp Ala Ser Glu Arg Thr Asn Tyr Thr Cys Cys
                325                 330                 335

Arg Leu Gln Arg His Glu Trp Asn Lys Leu Gly Trp Cys Asn Trp Tyr
            340                 345                 350

Asn Ile Asp Pro Trp Ile Gln Leu Met Asn Arg Thr Gln Thr Asn Leu
        355                 360                 365

Thr Glu Gly Pro Pro Asp Lys Glu Cys Ala Val Thr Cys Arg Tyr Asp
    370                 375                 380

Lys Asn Thr Asp Val Asn Val Val Thr Gln Ala Arg Asn Arg Pro Thr
385                 390                 395                 400

Thr Leu Thr Gly Cys Lys Lys Gly Lys Asn Phe Ser Phe Ala Gly Thr
                405                 410                 415

Val Ile Glu Gly Pro Cys Asn Phe Asn Val Ser Val Glu Asp Ile Leu
            420                 425                 430

Tyr Gly Asp His Glu Cys Gly Ser Leu Leu Gln Asp Thr Ala Leu Tyr
        435                 440                 445

Leu Leu Asp Gly Met Thr Asn Thr Ile Glu Asn Ala Arg Gln Gly Ala
    450                 455                 460

Ala Arg Val Thr Ser Trp Leu Gly Arg Gln Leu Ser Thr Ala Gly Lys
465                 470                 475                 480

Lys Leu Glu Arg Arg Ser Lys Thr Trp Phe Gly Ala Tyr Ala Leu
                485                 490                 495

SEQUENCE LISTING

(1) GENERAL INFORMATION:

(i) APPLICANT:
(A) NAME: Boehringer Ingelheim Vetmedica GmbH
(B) STREET: Binger Strasse 173
(C) CITY: Ingelheim
(E) COUNTRY: Germany
(F) POSTAL CODE (ZIP): 55216
(G) TELEPHONE: 06132777065
(H) TELEFAX: 06132774377

(ii) TITLE OF INVENTION: Attenuated pestiviruses

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98110356.7

(2) INFORMATION FOR SEQ ID NO: 1:

(i) SEQUENCE CHARACTERISTICS:
(A) LENGTH: 495 amino acids
(B) TYPE: amino acid
(C) STRÅNDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: internal

(vi) ORIGINAL SOURCE:
(A) ORGANISM: Pestivirus Glycoprotein Erns-297-K
(B) STRAIN: CSFV Classical Swine Fever Virus
(C) INDIVIDUAL ISOLATE: Alfort

(xi) SEQUENCE DESCRIPTION: SEQ ID NO: 1:

Met Glu Leu Asn His Phe Glu Leu Leu Tyr Lys Thr Ser Lys Gln Lys
1               5                   10                  15

Pro Val Gly Val Glu Glu Pro Val Tyr Asp Thr Ala Gly Arg Pro Leu
            20                  25                  30

Phe Gly Asn Pro Ser Glu Val His Pro Gln Ser Thr Leu Lys Leu Pro
        35                  40                  45

His Asp Arg Gly Arg Gly Asp Ile Arg Thr Thr Leu Arg Asp Leu Pro
    50                  55                  60

Arg Lys Gly Asp Cys Arg Ser Gly Asn His Leu Gly Pro Val Ser Gly
65                  70                  75                  80

Ile Tyr Ile Lys Pro Gly Pro Val Tyr Tyr Gln Asp Tyr Thr Gly Pro
                85                  90                  95

Val Tyr His Arg Ala Pro Leu Glu Phe Phe Asp Glu Ala Gln Phe Cys
            100                 105                 110

Glu Val Thr Lys Arg Ile Gly Arg Val Thr Gly Ser Asp Gly Lys Leu
        115                 120                 125

Tyr His Ile Tyr Val Cys Val Asp Gly Cys Ile Leu Leu Lys Leu Ala
    130                 135                 140

Lys Arg Gly Thr Pro Arg Thr Leu Lys Trp Ile Arg Asn Phe Thr Asn
145                 150                 155                 160

Cys Pro Leu Trp Val Thr Ser Cys Ser Asp Asp Gly Ala Ser Gly Ser
                165                 170                 175

Lys Asp Lys Lys Pro Asp Arg Met Asn Lys Gly Lys Leu Lys Ile Ala
            180                 185                 190

Pro Arg Glu His Glu Lys Asp Ser Lys Thr Lys Pro Pro Asp Ala Thr
        195                 200                 205

Ile Val Val Glu Gly Val Lys Tyr Gln Ile Lys Lys Lys Gly Lys Val
    210                 215                 220

Lys Gly Lys Asn Thr Gln Asp Gly Leu Tyr His Asn Lys Asn Lys Pro
225                 230                 235                 240

Pro Glu Ser Arg Lys Lys Leu Glu Lys Ala Leu Leu Ala Trp Ala Val
                245                 250                 255

Ile Thr Ile Leu Leu Tyr Gln Pro Val Ala Ala Glu Asn Ile Thr Gln
            260                 265                 270

Trp Asn Leu Ser Asp Asn Gly Thr Asn Gly Ile Gln Arg Ala Met Tyr
        275                 280                 285

Leu Arg Gly Val Asn Arg Ser Leu Lys Gly Ile Trp Pro Glu Lys Ile
    290                 295                 300

Cys Lys Gly Val Pro Thr His Leu Ala Thr Asp Thr Glu Leu Lys Glu
305                 310                 315                 320

Ile Arg Gly Met Met Asp Ala Ser Glu Arg Thr Asn Tyr Thr Cys Cys
                325                 330                 335

Arg Leu Gln Arg His Glu Trp Asn Lys His Gly Trp Cys Asn Trp Tyr
            340                 345                 350

Asn Ile Asp Pro Trp Ile Gln Leu Met Asn Arg Thr Gln Thr Asn Leu
        355                 360                 365

Thr Glu Gly Pro Pro Asp Lys Glu Cys Ala Val Thr Cys Arg Tyr Asp
    370                 375                 380

Lys Asn Thr Asp Val Asn Val Val Thr Gln Ala Arg Asn Arg Pro Thr
385                 390                 395                 400

Thr Leu Thr Gly Cys Lys Lys Gly Lys Asn Phe Ser Phe Ala Gly Thr
                405                 410                 415

Val Ile Glu Gly Pro Cys Asn Phe Asn Val Ser Val Glu Asp Ile Leu
            420                 425                 430

Tyr Gly Asp His Glu Cys Gly Ser Leu Leu Gln Asp Thr Ala Leu Tyr
        435                 440                 445

Leu Leu Asp Gly Met Thr Asn Thr Ile Glu Asn Ala Arg Gln Gly Ala
    450                 455                 460

Ala Arg Val Thr Ser Trp Leu Gly Arg Gln Leu Ser Thr Ala Gly Lys
465                 470                 475                 480

Lys Leu Glu Arg Arg Ser Lys Thr Trp Phe Gly Ala Tyr Ala Leu
                485                 490                 495

SEQUENCE LISTING

(1) GENERAL INFORMATION:

(i) APPLICANT:
(A) NAME: Boehringer Ingelheim Vetmedica GmbH
(B) STREET: Binger Strasse 173
(C) CITY: Ingelheim
(E) COUNTRY: Germany
(F) POSTAL CODE (ZIP): 55216
(G) TELEPHONE: 06132777065
(H) TELEFAX: 06132774377

(ii) TITLE OF INVENTION: Attenuated pestiviruses

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98110356.7

(2) INFORMATION FOR SEQ ID NO: 1:

(i) SEQUENCE CHARACTERISTICS:
(A) LENGTH: 495 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: internal

(vi) ORIGINAL SOURCE:
(A) ORGANISM: Pestivirus Glycoprotein Erns-346-K
(B) STRAIN: CSFV Classical Swine Fever Virus
(C) INDIVIDUAL ISOLATE: Alfort

(xi) SEQUENCE DESCRIPTION: SEQ ID NO: 1:

Met Glu Leu Asn His Phe Glu Leu Leu Tyr Lys Thr Ser Lys Gln Lys
1               5                   10                  15

Pro Val Gly Val Glu Glu Pro Val Tyr Asp Thr Ala Gly Arg Pro Leu
            20                  25                  30

Phe Gly Asn Pro Ser Glu Val His Pro Gln Ser Thr Leu Lys Leu Pro
        35                  40                  45

His Asp Arg Gly Arg Gly Asp Ile Arg Thr Thr Leu Arg Asp Leu Pro
    50                  55                  60

Arg Lys Gly Asp Cys Arg Ser Gly Asn His Leu Gly Pro Val Ser Gly
65                  70                  75                  80

Ile Tyr Ile Lys Pro Gly Pro Val Tyr Tyr Gln Asp Tyr Thr Gly Pro
                85                  90                  95

Val Tyr His Arg Ala Pro Leu Glu Phe Phe Asp Glu Ala Gln Phe Cys
            100                 105                 110

Glu Val Thr Lys Arg Ile Gly Arg Val Thr Gly Ser Asp Gly Lys Leu
        115                 120                 125

Tyr His Ile Tyr Val Cys Val Asp Gly Cys Ile Leu Leu Lys Leu Ala
    130                 135                 140

Lys Arg Gly Thr Pro Arg Thr Leu Lys Trp Ile Arg Asn Phe Thr Asn
145                 150                 155                 160

Cys Pro Leu Trp Val Thr Ser Cys Ser Asp Asp Gly Ala Ser Gly Ser
                165                 170                 175

Lys Asp Lys Lys Pro Asp Arg Met Asn Lys Gly Lys Leu Lys Ile Ala
            180                 185                 190

Pro Arg Glu His Glu Lys Asp Ser Lys Thr Lys Pro Pro Asp Ala Thr
        195                 200                 205

Ile Val Val Glu Gly Val Lys Tyr Gln Ile Lys Lys Lys Gly Lys Val
    210                 215                 220

Lys Gly Lys Asn Thr Gln Asp Gly Leu Tyr His Asn Lys Asn Lys Pro
225                 230                 235                 240

Pro Glu Ser Arg Lys Lys Leu Glu Lys Ala Leu Leu Ala Trp Ala Val
                245                 250                 255

Ile Thr Ile Leu Leu Tyr Gln Pro Val Ala Ala Glu Asn Ile Thr Gln
            260                 265                 270

Trp Asn Leu Ser Asp Asn Gly Thr Asn Gly Ile Gln Arg Ala Met Tyr
        275                 280                 285

Leu Arg Gly Val Asn Arg Ser Leu His Gly Ile Trp Pro Glu Lys Ile
    290                 295                 300

Cys Lys Gly Val Pro Thr His Leu Ala Thr Asp Thr Glu Leu Lys Glu
305                 310                 315                 320

Ile Arg Gly Met Met Asp Ala Ser Glu Arg Thr Asn Tyr Thr Cys Cys
                325                 330                 335

Arg Leu Gln Arg His Glu Trp Asn Lys Lys Gly Trp Cys Asn Trp Tyr
            340                 345                 350

Asn Ile Asp Pro Trp Ile Gln Leu Met Asn Arg Thr Gln Thr Asn Leu
        355                 360                 365

Thr Glu Gly Pro Pro Asp Lys Glu Cys Ala Val Thr Cys Arg Tyr Asp
    370                 375                 380

Lys Asn Thr Asp Val Asn Val Val Thr Gln Ala Arg Asn Arg Pro Thr
385                 390                 395                 400

Thr Leu Thr Gly Cys Lys Lys Gly Lys Asn Phe Ser Phe Ala Gly Thr
                405                 410                 415

Val Ile Glu Gly Pro Cys Asn Phe Asn Val Ser Val Glu Asp Ile Leu
            420                 425                 430

Tyr Gly Asp His Glu Cys Gly Ser Leu Leu Gln Asp Thr Ala Leu Tyr
        435                 440                 445

Leu Leu Asp Gly Met Thr Asn Thr Ile Glu Asn Ala Arg Gln Gly Ala
    450                 455                 460

Ala Arg Val Thr Ser Trp Leu Gly Arg Gln Leu Ser Thr Ala Gly Lys
465                 470                 475                 480

Lys Leu Glu Arg Arg Ser Lys Thr Trp Phe Gly Ala Tyr Ala Leu
                485                 490                 495

SEQUENCE LISTING

(1) GENERAL INFORMATION:

(i) APPLICANT:
(A) NAME: Boehringer Ingelheim Vetmedica GmbH
(B) STREET: Binger Strasse 173
(C) CITY: Ingelheim
(E) COUNTRY: Germany
(F) POSTAL CODE (ZIP): 55216
(G) TELEPHONE: 06132777065
(H) TELEFAX: 06132774377

(ii) TITLE OF INVENTION: Attenuated pestiviruses

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98110356.7

(2) INFORMATION FOR SEQ ID NO: 1:

(i) SEQUENCE CHARACTERISTICS:
(A) LENGTH: 495 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: internal

(vi) ORIGINAL SOURCE:
(A) ORGANISM: Pestivirus Glycoprotein Erns
(B) STRAIN: CSFV Classical Swine Fever Virus-297-K-346-K
(C) INDIVIDUAL ISOLATE: Alfort

(xi) SEQUENCE DESCRIPTION: SEQ ID NO: 1:

Met Glu Leu Asn His Phe Glu Leu Leu Tyr Lys Thr Ser Lys Gln Lys
1               5                   10                  15

Pro Val Gly Val Glu Glu Pro Val Tyr Asp Thr Ala Gly Arg Pro Leu
            20                  25                  30

Phe Gly Asn Pro Ser Glu Val His Pro Gln Ser Thr Leu Lys Leu Pro
        35                  40                  45

His Asp Arg Gly Arg Gly Asp Ile Arg Thr Thr Leu Arg Asp Leu Pro
    50                  55                  60

Arg Lys Gly Asp Cys Arg Ser Gly Asn His Leu Gly Pro Val Ser Gly
65                  70                  75                  80

Ile Tyr Ile Lys Pro Gly Pro Val Tyr Tyr Gln Asp Tyr Thr Gly Pro
                85                  90                  95

Val Tyr His Arg Ala Pro Leu Glu Phe Phe Asp Glu Ala Gln Phe Cys
            100                 105                 110

Glu Val Thr Lys Arg Ile Gly Arg Val Thr Gly Ser Asp Gly Lys Leu
        115                 120                 125

Tyr His Ile Tyr Val Cys Val Asp Gly Cys Ile Leu Leu Lys Leu Ala
    130                 135                 140

Lys Arg Gly Thr Pro Arg Thr Leu Lys Trp Ile Arg Asn Phe Thr Asn
145                 150                 155                 160

Cys Pro Leu Trp Val Thr Ser Cys Ser Asp Asp Gly Ala Ser Gly Ser
                165                 170                 175

Lys Asp Lys Lys Pro Asp Arg Met Asn Lys Gly Lys Leu Lys Ile Ala
            180                 185                 190

Pro Arg Glu His Glu Lys Asp Ser Lys Thr Lys Pro Pro Asp Ala Thr
        195                 200                 205

Ile Val Val Glu Gly Val Lys Tyr Gln Ile Lys Lys Lys Gly Lys Val
    210                 215                 220

Lys Gly Lys Asn Thr Gln Asp Gly Leu Tyr His Asn Lys Asn Lys Pro
225                 230                 235                 240

Pro Glu Ser Arg Lys Lys Leu Glu Lys Ala Leu Leu Ala Trp Ala Val
                245                 250                 255

Ile Thr Ile Leu Leu Tyr Gln Pro Val Ala Ala Glu Asn Ile Thr Gln
            260                 265                 270

Trp Asn Leu Ser Asp Asn Gly Thr Asn Gly Ile Gln Arg Ala Met Tyr
        275                 280                 285

Leu Arg Gly Val Asn Arg Ser Leu Lys Gly Ile Trp Pro Glu Lys Ile
    290                 295                 300

Cys Lys Gly Val Pro Thr His Leu Ala Thr Asp Thr Glu Leu Lys Glu
305                 310                 315                 320

Ile Arg Gly Met Met Asp Ala Ser Glu Arg Thr Asn Tyr Thr Cys Cys
                325                 330                 335

Arg Leu Gln Arg His Glu Trp Asn Lys Lys Gly Trp Cys Asn Trp Tyr
            340                 345                 350

Asn Ile Asp Pro Trp Ile Gln Leu Met Asn Arg Thr Gln Thr Asn Leu
        355                 360                 365

Thr Glu Gly Pro Pro Asp Lys Glu Cys Ala Val Thr Cys Arg Tyr Asp
    370                 375                 380

Lys Asn Thr Asp Val Asn Val Val Thr Gln Ala Arg Asn Arg Pro Thr
385                 390                 395                 400

Thr Leu Thr Gly Cys Lys Lys Gly Lys Asn Phe Ser Phe Ala Gly Thr
                405                 410                 415

Val Ile Glu Gly Pro Cys Asn Phe Asn Val Ser Val Glu Asp Ile Leu
            420                 425                 430

Tyr Gly Asp His Glu Cys Gly Ser Leu Leu Gln Asp Thr Ala Leu Tyr
        435                 440                 445

Leu Leu Asp Gly Met Thr Asn Thr Ile Glu Asn Ala Arg Gln Gly Ala
    450                 455                 460

Ala Arg Val Thr Ser Trp Leu Gly Arg Gln Leu Ser Thr Ala Gly Lys
465                 470                 475                 480

Lys Leu Glu Arg Arg Ser Lys Thr Trp Phe Gly Ala Tyr Ala Leu
                485                 490                 495

SEQUENCE LISTING

(1) GENERAL INFORMATION:

(i) APPLICANT:
(A) NAME: Boehringer Ingelheim Vetmedica GmbH
(B) STREET: Binger Strasse 173
(C) CITY: Ingelheim
(E) COUNTRY: Germany
(F) POSTAL CODE (ZIP): 55216
(G) TELEPHONE: 06132777065
(H) TELEFAX: 06132774377

(ii) TITLE OF INVENTION: Attenuated Pestiviruses

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98110356.7

(2) INFORMATION FOR SEQ ID NO: 1:

(i) SEQUENCE CHARACTERISTICS:
(A) LENGTH: 494 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: internal

(vi) ORIGINAL SOURCE:
(A) ORGANISM: Pestivirus Glycoprotein Erns
(B) STRAIN: CSFV Classical Swine Fever Virus-346-d
(C) INDIVIDUAL ISOLATE: Alfort

(xi) SEQUENCE DESCRIPTION: SEQ ID NO: 1:

Met Glu Leu Asn His Phe Glu Leu Leu Tyr Lys Thr Ser Lys Gln Lys
1               5                   10                  15

Pro Val Gly Val Glu Glu Pro Val Tyr Asp Thr Ala Gly Arg Pro Leu
            20                  25                  30

Phe Gly Asn Pro Ser Glu Val His Pro Gln Ser Thr Leu Lys Leu Pro
        35                  40                  45

His Asp Arg Gly Arg Gly Asp Ile Arg Thr Thr Leu Arg Asp Leu Pro
    50                  55                  60

Arg Lys Gly Asp Cys Arg Ser Gly Asn His Leu Gly Pro Val Ser Gly
65                  70                  75                  80

Ile Tyr Ile Lys Pro Gly Pro Val Tyr Tyr Gln Asp Tyr Thr Gly Pro
                85                  90                  95

Val Tyr His Arg Ala Pro Leu Glu Phe Phe Asp Glu Ala Gln Phe Cys
            100                 105                 110

Glu Val Thr Lys Arg Ile Gly Arg Val Thr Gly Ser Asp Gly Lys Leu
        115                 120                 125

Tyr His Ile Tyr Val Cys Val Asp Gly Cys Ile Leu Leu Lys Leu Ala
    130                 135                 140

Lys Arg Gly Thr Pro Arg Thr Leu Lys Trp Ile Arg Asn Phe Thr Asn
145                 150                 155                 160

Cys Pro Leu Trp Val Thr Ser Cys Ser Asp Asp Gly Ala Ser Gly Ser
                165                 170                 175

Lys Asp Lys Lys Pro Asp Arg Met Asn Lys Gly Lys Leu Lys Ile Ala
            180                 185                 190

Pro Arg Glu His Glu Lys Asp Ser Lys Thr Lys Pro Pro Asp Ala Thr
        195                 200                 205

Ile Val Val Glu Gly Val Lys Tyr Gln Ile Lys Lys Lys Gly Lys Val
    210                 215                 220

Lys Gly Lys Asn Thr Gln Asp Gly Leu Tyr His Asn Lys Asn Lys Pro
225                 230                 235                 240

Pro Glu Ser Arg Lys Lys Leu Glu Lys Ala Leu Leu Ala Trp Ala Val
                245                 250                 255

Ile Thr Ile Leu Leu Tyr Gln Pro Val Ala Ala Glu Asn Ile Thr Gln
            260                 265                 270

Trp Asn Leu Ser Asp Asn Gly Thr Asn Gly Ile Gln Arg Ala Met Tyr
        275                 280                 285

Leu Arg Gly Val Asn Arg Ser Leu His Gly Ile Trp Pro Glu Lys Ile
    290                 295                 300

Cys Lys Gly Val Pro Thr His Leu Ala Thr Asp Thr Glu Leu Lys Glu
305                 310                 315                 320

Ile Arg Gly Met Met Asp Ala Ser Glu Arg Thr Asn Tyr Thr Cys Cys
                325                 330                 335

Arg Leu Gln Arg His Glu Trp Asn Lys Gly Trp Cys Asn Trp Tyr Asn
            340                 345                 350

Ile Asp Pro Trp Ile Gln Leu Met Asn Arg Thr Gln Thr Asn Leu Thr
        355                 360                 365

Glu Gly Pro Pro Asp Lys Glu Cys Ala Val Thr Cys Arg Tyr Asp Lys
    370                 375                 380

Asn Thr Asp Val Asn Val Val Thr Gln Ala Arg Asn Arg Pro Thr Thr
385                 390                 395                 400

Leu Thr Gly Cys Lys Lys Gly Lys Asn Phe Ser Phe Ala Gly Thr Val
                405                 410                 415

Ile Glu Gly Pro Cys Asn Phe Asn Val Ser Val Glu Asp Ile Leu Tyr
            420                 425                 430

Gly Asp His Glu Cys Gly Ser Leu Leu Gln Asp Thr Ala Leu Tyr Leu
        435                 440                 445

Leu Asp Gly Met Thr Asn Thr Ile Glu Asn Ala Arg Gln Gly Ala Ala
    450                 455                 460

Arg Val Thr Ser Trp Leu Gly Arg Gln Leu Ser Thr Ala Gly Lys Lys
465                 470                 475                 480

Leu Glu Arg Arg Ser Lys Thr Trp Phe Gly Ala Tyr Ala Leu
                485                 490

SEQUENCE LISTING

(1) GENERAL INFORMATION:

(i) APPLICANT:
(A) NAME: Boehringer Ingelheim Vetmedica GmbH
(B) STREET: Binger Strasse 173
(C) CITY: Ingelheim
(E) COUNTRY: Germany
(F) POSTAL CODE (ZIP): 55216
(G) TELEPHONE: 06132777065
(H) TELEFAX: 06132774377

(ii) TITLE OF INVENTION: Attenuated pestiviruses

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98110356.7

(2) INFORMATION FOR SEQ ID NO: 1:

(i) SEQUENCE CHARACTERISTICS:
(A) LENGTH: 495 amino acids
(B) TYPE: amino acid
(C) STRÅNDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: internal

(vi) ORIGINAL SOURCE:
(A) ORGANISM: Pestivirus Glycoprotein Erns
(B) STRAIN: BVDV Bovine Viral Diarrhea Virus
(C) INDIVIDUAL ISOLATE: CP7

(xi) SEQUENCE DESCRIPTION: SEQ ID NO: 1:

Met Glu Leu Ile Thr Asn Glu Leu Leu Tyr Lys Thr Tyr Lys Gln Lys
1               5                   10                  15

Pro Ala Gly Val Glu Glu Pro Val Tyr Asp Gln Ala Gly Asn Pro Leu
            20                  25                  30

Phe Gly Glu Arg Gly Val Ile His Pro Gln Ser Thr Leu Lys Leu Pro
        35                  40                  45

His Lys Arg Gly Glu Arg Glu Val Pro Thr Asn Leu Ala Ser Leu Pro
    50                  55                  60

Lys Arg Gly Asp Cys Arg Ser Gly Asn Ser Lys Gly Pro Val Ser Gly
65                  70                  75                  80

Ile Tyr Leu Lys Pro Gly Pro Leu Phe Tyr Gln Asp Tyr Lys Gly Pro
                85                  90                  95

Val Tyr His Arg Ala Pro Leu Glu Phe Phe Glu Glu Ala Ser Met Cys
            100                 105                 110

Glu Thr Thr Lys Arg Ile Gly Arg Val Thr Gly Ser Asp Ser Arg Leu
        115                 120                 125

Tyr His Ile Tyr Val Cys Ile Asp Gly Cys Ile Ile Val Lys Ser Ala
    130                 135                 140

Thr Lys Asp Arg Gln Lys Val Leu Lys Trp Val His Asn Lys Leu Asn
145                 150                 155                 160

Cys Pro Leu Trp Val Ser Ser Cys Ser Asp Thr Lys Asp Glu Gly Val
                165                 170                 175

Val Arg Lys Lys Gln Gln Lys Pro Asp Arg Leu Glu Lys Gly Arg Met
            180                 185                 190

Lys Ile Thr Pro Lys Glu Ser Glu Lys Asp Ser Lys Thr Lys Pro Pro
        195                 200                 205

Asp Ala Thr Ile Val Val Asp Gly Val Lys Tyr Gln Val Lys Lys Lys
    210                 215                 220

Gly Lys Val Lys Ser Lys Asn Thr Gln Asp Gly Leu Tyr His Asn Lys
225                 230                 235                 240

Asn Lys Pro Gln Glu Ser Arg Lys Lys Leu Glu Lys Ala Leu Leu Ala
                245                 250                 255

Trp Ala Ile Ile Ala Leu Val Phe Phe Gln Val Thr Met Gly Glu Asn
            260                 265                 270

Ile Thr Gln Trp Asn Leu Gln Asp Asn Gly Thr Glu Gly Ile Gln Arg
        275                 280                 285

Ala Met Phe Gln Arg Gly Val Asn Arg Ser Leu His Gly Ile Trp Pro
    290                 295                 300

Glu Lys Ile Cys Thr Gly Val Pro Ser His Leu Ala Thr Asp Thr Glu
305                 310                 315                 320

Leu Lys Ala Ile His Gly Met Met Asp Ala Ser Glu Lys Thr Asn Tyr
                325                 330                 335

Thr Cys Cys Arg Leu Gln Arg His Glu Trp Asn Lys His Gly Trp Cys
            340                 345                 350

Asn Trp Tyr Asn Ile Glu Pro Trp Ile Leu Leu Met Asn Lys Thr Gln
        355                 360                 365

Ala Asn Leu Thr Glu Gly Gln Pro Leu Arg Glu Cys Ala Val Thr Cys
    370                 375                 380

Arg Tyr Asp Arg Asp Ser Asp Leu Asn Val Val Thr Gln Ala Arg Asp
385                 390                 395                 400

Ser Pro Thr Pro Leu Thr Gly Cys Lys Lys Gly Lys Asn Phe Ser Phe
                405                 410                 415

Ala Gly Ile Leu Val Gln Gly Pro Cys Asn Phe Glu Ile Ala Val Ser
            420                 425                 430

Asp Val Leu Phe Lys Glu His Asp Cys Thr Ser Val Ile Gln Asp Thr
        435                 440                 445

Ala His Tyr Leu Val Asp Gly Met Thr Asn Ser Leu Glu Ser Ala Arg
    450                 455                 460

Gln Gly Thr Ala Lys Leu Thr Thr Trp Leu Gly Arg Gln Leu Gly Ile
465                 470                 475                 480

Leu Gly Lys Lys Leu Glu Asn Lys Ser Lys Thr Trp Phe Gly Ala
                485                 490                 495

SEQUENCE LISTING

(1) GENERAL INFORMATION:

(i) APPLICANT:
(A) NAME: Boehringer Ingelheim Vetmedica GmbH
(B) STREET: Binger Strasse 173
(C) CITY: Ingelheim
(E) COUNTRY: Germany
(F) POSTAL CODE (ZIP): 55216
(G) TELEPHONE: 06132777065
(H) TELEFAX: 06132774377

(ii) TITLE OF INVENTION: Attenuated pestiviruses

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98110356.7

(2) INFORMATION FOR SEQ ID NO: 1:

(i) SEQUENCE CHARACTERISTICS:
(A) LENGTH: 494 amino acids
(B) TYPE: amino acid
(C) STRÅNDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: internal

(vi) ORIGINAL SOURCE:
(A) ORGANISM: Pestivirus Glycoprotein Erns
(B) STRAIN: BVDV Bovine Viral Diarrhea Virus -346-d (349)
(C) INDIVIDUAL ISOLATE: CP7

(xi) SEQUENCE DESCRIPTION: SEQ ID NO: 1:

Met Glu Leu Ile Thr Asn Glu Leu Leu Tyr Lys Thr Tyr Lys Gln Lys
1               5                   10                  15

Pro Ala Gly Val Glu Glu Pro Val Tyr Asp Gln Ala Gly Asn Pro Leu
            20                  25                  30

Phe Gly Glu Arg Gly Val Ile His Pro Gln Ser Thr Leu Lys Leu Pro
        35                  40                  45

His Lys Arg Gly Glu Arg Glu Val Pro Thr Asn Leu Ala Ser Leu Pro
    50                  55                  60

Lys Arg Gly Asp Cys Arg Ser Gly Asn Ser Lys Gly Pro Val Ser Gly
65                  70                  75                  80

Ile Tyr Leu Lys Pro Gly Pro Leu Phe Tyr Gln Asp Tyr Lys Gly Pro
                85                  90                  95

Val Tyr His Arg Ala Pro Leu Glu Phe Phe Glu Glu Ala Ser Met Cys
            100                 105                 110

Glu Thr Thr Lys Arg Ile Gly Arg Val Thr Gly Ser Asp Ser Arg Leu
        115                 120                 125

Tyr His Ile Tyr Val Cys Ile Asp Gly Cys Ile Ile Val Lys Ser Ala
    130                 135                 140

Thr Lys Asp Arg Gln Lys Val Leu Lys Trp Val His Asn Lys Leu Asn
145                 150                 155                 160

Cys Pro Leu Trp Val Ser Ser Cys Ser Asp Thr Lys Asp Glu Gly Val
                165                 170                 175

Val Arg Lys Lys Gln Gln Lys Pro Asp Arg Leu Glu Lys Gly Arg Met
            180                 185                 190

Lys Ile Thr Pro Lys Glu Ser Glu Lys Asp Ser Lys Thr Lys Pro Pro
        195                 200                 205

Asp Ala Thr Ile Val Val Asp Gly Val Lys Tyr Gln Val Lys Lys Lys
    210                 215                 220

Gly Lys Val Lys Ser Lys Asn Thr Gln Asp Gly Leu Tyr His Asn Lys
225                 230                 235                 240

Asn Lys Pro Gln Glu Ser Arg Lys Lys Leu Glu Lys Ala Leu Leu Ala
                245                 250                 255

Trp Ala Ile Ile Ala Leu Val Phe Phe Gln Val Thr Met Gly Glu Asn
            260                 265                 270

Ile Thr Gln Trp Asn Leu Gln Asp Asn Gly Thr Glu Gly Ile Gln Arg
        275                 280                 285

Ala Met Phe Gln Arg Gly Val Asn Arg Ser Leu His Gly Ile Trp Pro
    290                 295                 300

Glu Lys Ile Cys Thr Gly Val Pro Ser His Leu Ala Thr Asp Thr Glu
305                 310                 315                 320

Leu Lys Ala Ile His Gly Met Met Asp Ala Ser Glu Lys Thr Asn Tyr
                325                 330                 335

Thr Cys Cys Arg Leu Gln Arg His Glu Trp Asn Lys Gly Trp Cys Asn
            340                 345                 350

Trp Tyr Asn Ile Glu Pro Trp Ile Leu Leu Met Asn Lys Thr Gln Ala
        355                 360                 365

Asn Leu Thr Glu Gly Gln Pro Leu Arg Glu Cys Ala Val Thr Cys Arg
    370                 375                 380

Tyr Asp Arg Asp Ser Asp Leu Asn Val Val Thr Gln Ala Arg Asp Ser
385                 390                 395                 400

Pro Thr Pro Leu Thr Gly Cys Lys Lys Gly Lys Asn Phe Ser Phe Ala
                405                 410                 415

Gly Ile Leu Val Gln Gly Pro Cys Asn Phe Glu Ile Ala Val Ser Asp
            420                 425                 430

Val Leu Phe Lys Glu His Asp Cys Thr Ser Val Ile Gln Asp Thr Ala
        435                 440                 445

His Tyr Leu Val Asp Gly Met Thr Asn Ser Leu Glu Ser Ala Arg Gln
    450                 455                 460

Gly Thr Ala Lys Leu Thr Thr Trp Leu Gly Arg Gln Leu Gly Ile Leu
465                 470                 475                 480

Gly Lys Lys Leu Glu Asn Lys Ser Lys Thr Trp Phe Gly Ala
                485                 490